# EUROPEAN PATENT SPECIFICATION

(11) **EP 3 729 062 B1**
(45) Date of publication and mention of the grant of the patent: **17.08.2022**
(21) Application number: 18819270.2
(22) Date of filing: 10.12.2018
(51) Int. Cl.: G01N 21/78, G01N 21/84, G01N 33/558, G01N 21/77

(54) **LATERAL FLOW DIAGNOSTIC DEVICE**
LATERAL-FLOW-DIAGNOSEGERÄT
DISPOSITIF DE DIAGNOSTIQUE À L'ÉCOULEMENT LATÉRAL

(30) Priority: 22.12.2017 GB 201721796
(43) Date of publication of application: 28.10.2020
(73) Proprietor: University of Southampton, Southampton, Hampshire SO17 1BJ (GB)
(72) Inventor: EASON, Robert William, Southampton SO17 1BJ (GB); SONES, Collin Lawrence, Southampton SO17 1BJ (GB); KATIS, Ioannis Nikolaos, Southampton SO17 1BJ (GB); HE, Peijun, Southampton SO17 1BJ (GB)
(74) Representative: D Young & Co LLP
(86) International application number: PCT/GB2018/053576
(87) International publication number: WO 2019/122816

(56) References cited:
- WO-A1-2016/178013
- CN-A- 106 501 509
- CN-A- 107 367 609
- US-A1- 2017 074 874
- US-B1- 6 656 745

## Description

The present technique relates to the field of lateral flow diagnostic devices.

Point-of-care (POC) rapid diagnostics have seen tremendous development in recent years, as the emphasis of care is shifting towards prevention and early detection of disease. Such testing gives immediate results in non-laboratory settings, do not require any technical skills from the user and are relatively inexpensive, making them ideal for use not only in poorly-resourced, remote areas of developing countries, but also in hospitals and at the bedside of well-developed nations. One example of such POC diagnostics is paper-based fluidic devices. A subcategory of POC diagnostics, lateral flow devices (LFDs), are currently the primary means for the detection of a variety of analytes and the most common example of such devices is the pregnancy test that can be easily bought over the counter at a pharmacy or local shop. These are POC devices in their simplest form and even though their range of applications has been widely expanded to detection of other diseases or conditions, there has not been any other major advancement to their capabilities, therefore limiting their clinical uptake. They possess many intrinsic advantages such as low-cost, capability of mass production, are disposable, equipment free, and require no external power to operate. LFDs can also be useful for non-medical diagnostic applications, such as detection of analytes in fluid samples for food, beverage, pharmaceutical or water industries, environmental sensing, etc.

CN106501509 A describes a high-sensitivity enriching immunochromatography test paper strip The high-sensitivity enriching chromatography test paper strip mainly comprises a sample pad, a colloidal gold pad, a chromatography membrane, a water absorption pad and a backer board, wherein the chromatography membrane is of a structure with a narrow middle and two wide sides, a detection zone is located at the narrow position of the chromatography membrane, and a quality control zone is located above the detection zone. Gold nanoparticles labelled by a detection antibody are bonded on the colloidal gold pad, the detection zone is coated with a captured antibody or antigen, and the quality control zone is coated with a second antibody. The enriching immunochromatography test paper strip rapidly enriches to-be-detected ingredients in a sample by arranging a narrow zone, the operation is simple, the detection sensitivity of the sample is greatly improved, detected objects can be protein, nucleic acid, sugar and other bio-macromolecular and micromolecular compounds and cellular and subcellular ingredients, and the high-sensitivity enriching chromatography test paper strip can be used in the fields of clinical diagnosis, inspection and quarantine, food safety inspection, judicial expertise, drug detection and the like. US6656745 B1 describes an immunoassay device featuring a semi-quantitative, multi-level method for determining the presence and an amount of an analyte. The immunoassay involves a device comprising a layer with a porous medium deposited thereon. The device has several binding zones in which each zone comprises a defined amount of a binding reagent to bind and immobilize the analyte. The device also features an inscribed capillary channel having fluid barriers that allow control of a rate of migration of fluid along the porous medium. The device typically provides at least some of the binding zones within the capillary channels such that a solvent quickly traverses through the capillary channels and provides a semi-quantitative result. Preferably, any binding of an analyte is accompanied by a visual change in the binding zone such that one can easily detect visually an amount of analyte present in the sample. It also describes a method for determining the presence and amount of an analyte. It also describes a device for facilitating removal of liquids and/or preventing back flow along a porous medium comprising a series of capillary channels in parallel arrangement with each other in which each of the capillary channels are contiguous with the fluid pathway. It also describes a method for making an immunoassay device comprising providing a layer of a continuous porous medium and etching the medium to provide fluid barriers, at least a portion of which define a capillary channel.

WO2016/178013 A1 describes a method of making a fluid flow device comprising providing a substrate of porous material, depositing a radiation-sensitive substance onto the substrate in a pattern defining one or more regions intended to receive and contain fluid during use of the device or occupying an area within such a region, such that the radiation-sensitive substance extends at least partly through the thickness of the substrate below the pattern, and exposing radiation onto the substrate thereby delivering energy to the radiation-sensitive substance in at least part of the pattern to change the radiation-sensitive substance from a first state to a second state through at least part of the thickness of the substrate. One of the first state and the second state may be less permeable than the other.

US2017/074874 A1 describes lateral flow immunoassay systems, devices and methods, for detecting analytes in biological samples. More specifically, it relates to synthetic thread based lateral flow immunofluorescent assay systems, devices and methods. The lateral flow immunofluorescent assay devices can comprise one or more synthetic polymer threads defining at least a sample loading zone, a detection zone comprising an immobilised capture reagent that has affinity for a predetermined analyte in the sample, and an intermediate zone disposed between the sample loading zone and the detection zone, the intermediate zone comprising a fluorescent detection reagent for use in binding to a predetermined analyte in the sample to form a fluorescently labelled analyte, wherein the fluorescent detection reagent comprises fluorescently labelled microparticles that are associated, linked or coordinated to an analyte binding reagent that has affinity for a predetermined analyte in the sample, and wherein the one or more synthetic polymer threads are capable of carrying a fluid sample by capillary action from at least the sample loading zone to the detection zone.

CN107367609 A describes a lateral flow testing sheet and a detection method thereof. The lateral flow testing sheet comprises a sample pad, a bonding zone, a first quality control zone, a detection zone, a second quality control zone and an absorption pad, wherein the first quality control zone is a narrowing zone and has a radial width d1, the detection zone is a narrow zone and has a radial width d2, the second quality control zone is an enlargement zone and has a radial width d3, the radial width of the binding zone is d0, the radial width of the absorption pad is d4, the mean value of d4 is more than the mean value of d3, the mean value of d3 is more than the mean value of d2, the mean value of d0 is more than the mean value of d1, and the mean value of d1 is more than the mean value of d2.

According to the invention a lateral flow diagnostic device according to claim 1 and a method of manufacturing of said lateral flow diagnostic device according to claim 12 is provided.

Further aspects, features and advantages of the present technique will be apparent from the following description of examples, which is to be read in conjunction with the accompanying drawings, in which:
Figure 1 illustrates a schematic representation of a sandwich assay;
Figure 2 schematically illustrates two examples, not falling within the scope of the invention, of a lateral flow diagnostic device (LFD), one with a fluid flow channel of constant cross section, and another with a fluid flow channel having a constricted portion with a smaller cross-sectional area than other portions of the flow channel;
Figure 3 is a graph showing how the flow time taken for fluid to pass through the LFD varies with constriction width;
Figure 4 shows how the flow time for fluid to pass through the constricted portion varies with constriction width;
Figure 5 shows the variation in test line area with constriction width;
Figure 6 shows scanned images of LFDs with different constriction widths showing the test line colour intensity at different analyte concentrations;
Figure 7 is a graph showing the effect of varying constriction width on colour intensity measurements for different concentrations of analyte;
Figure 8 shows how the ratio between the colour intensity measured at constriction width of 1 mm and the colour intensity measured at a constriction width of 5 mm varies over a range of analyte concentrations;
Figure 9 illustrates how the limit of detection for a lateral flow assay varies depending on the constriction width;
Figure 10 illustrates calibration curves and dynamic ranges for fluid flow channels with different widths;
Figures 11 and 12 show examples of LFDs according to the invention with multiple fluid flow channels with test sites using the same analyte detection substance, but with different channel widths in the portion comprising the test site;
Figure 13 schematically illustrates a system for manufacturing the LFD;
Figure 14 schematically illustrates a method of forming fluid flow structures by applying radiation to convert a radiation-sensitive substance from the first state or second state;
Figure 15 shows an illustration of a laser directed write technique in which radiation-sensitive substance is selectively deposited by a deposition head; and
Figure 16 illustrates an example of an LFD, not falling within the scope of the invention, in which the constricted portion of the channel is constricted in the thickness dimension.

A lateral flow diagnostic device (lateral flow device, or LFD) may comprise a substrate of porous material, and channel boundaries which define a fluid flow channel within the substrate. The channel boundaries may include the edge(s) of the substrate, and/or could include structures of less porous material than the substrate itself, which are formed within the body of the substrate. A test site may be disposed along part of the fluid flow channel. The test site comprises an analyte detection substance for detecting the presence of a target analyte in fluid flowing along the flow channel. For example, the analyte detection substance may be a substance which reacts with the target analyte to produce a colour change at the test site, which can be visible to the user to provide an indication of whether the target analyte has been detected.

A disadvantage with typical LFDs is that they typically have a relatively high limit of detection, so they have been restricted for use with samples having highly abundant analyte. It would be desirable to enable them to be used with samples with lower analyte concentrations. A factor that determines the limit of detection and sensitivity of a lateral flow assay is the reaction time between the sample and the analyte detection substance deposited at the test site, which depends on the migration time of the sample over the test line. In the techniques discussed below, an LFD has a fluid flow channel having a constricted portion with a smaller cross-sectional area than another portion of the fluid flow channel. The test site is disposed within the constricted portion of the fluid flow channel. The use of such a constriction helps to reduce the limit of detection for several reasons. Firstly, the constriction in the fluid flow channel reduces the flow rate of fluid over the test site, increasing the reaction time between the sample and target analyte, to increase the likelihood that, even if the concentration of the target analyte is relatively low, the reaction will produce a visible result at the test site. Secondly, the constriction in the flow channel concentrates the target analyte into a smaller area, effectively increasing the concentration of target analyte seen by the test site, to increase the likelihood that the reaction at the test site can generate an observable result.

One may think that simply producing a fluid flow channel with a reduced cross-sectional area along its entire length would produce the same effect. However, this would be incorrect, as a narrower channel would mean that the total flow time taken to traverse the fluid flow channel would be increased, which may increase the length of time before the test result is observable at the test site. This can reduce the ease of use of the device, as the user may typically expect a fast test response and so needs the result to be visible as soon as possible. Also, if the entire channel has a reduced cross sectional area then the total volume of sample which can be processed by the LFD would be reduced, hence for a given analyte concentration the total amount of target analyte present would also be reduced, so that the channel would not produce the same reduction in the limit of sensitivity. In contrast, by providing a fluid flow channel with at least one portion with a larger cross sectional area and a constricted portion with a smaller cross sectional area, and disposing the test site within the constricted portion, this enables a greater volume of sample to be processed and flow rates in parts of the channel other than the constricted portion to be kept higher.

The fluid flow channel may have a fluid inlet for introducing fluid into the fluid flow channel. For example, a sample absorption pad may be provided at the fluid inlet. To supply the fluid sample into the fluid flow channel, the absorption pad can be dipped into the sample to soak the pad in the sample, from where the fluid may pass into and through the fluid flow channel. An initial portion of the fluid flow channel disposed between the fluid inlet and the constriction portion may have a larger cross-sectional area than the constricted portion. It can be particularly advantageous to have a larger cross sectional area in the initial part of the fluid flow channel, before then reducing the cross sectional area at the entry to the constricted portion which contains the test site. This means the time taken to flow from the fluid inlet to the entry region of the constricted portion can be kept shorter by having a wider cross sectional area in the initial portion, so that the user can view the result of the test sooner than if the cross sectional area in the initial portion was smaller. Also, the narrowing of the cross sectional area at the entry to the constricted portion concentrates the amount of target analyte provided into a smaller area, increasing the concentration of the target analyte and hence the reaction rate with the analyte detection substance at the test site. Therefore, the limit of detection can be reduced without greatly slowing down the overall time to obtain a result.

In some examples, once the fluid has flowed beyond the test site then the cross sectional area could then remain at the smaller cross sectional area so that the constricted portion of the flow channel extends all the way to the end of the fluid flow channel. However, in other examples a subsequent portion of the fluid flow channel lying beyond the constricted portion in a direction of fluid flow through the channel may have a larger cross sectional area than constricted portion. By increasing the cross sectional area once the fluid has passed beyond the test site, this again enables a larger quantity of sample to be retained beyond the test site before the channel becomes saturated. By enabling the LFD to work with larger volumes of fluid sample, this prevents the channel becoming saturated as soon, effectively increasing, for a given concentration of the target analyte, the total quantity of target analyte exposed to the test site. Hence, the limit of sensitivity can be decreased, as it is more likely that the test will produce an observable result even if the concentration of the target analyte is lower.

In addition to the test site, the device may also comprise a control site comprising a control substance for detecting presence of a substance indicative of validity of a test performed at the test site. For example, a conjugate pad may be provided at the inlet to the fluid flow channel, impregnated with a conjugate substance which will pass down the channel along with the sample itself when the sample is supplied. The conjugate substance (or the conjugate substance when bound to molecules within the sample) may trigger a reaction of the control substance which produces an observable result (such as a colour change). The control site may be disposed further away from the fluid inlet than the test site, so that the sample has to travel past the test site to reach the control site. Hence, regardless of whether the target analyte is present, when the sample is supplied into the channel, an observable change at the control site may indicate that the sample travelled past the test site and so the test has been validly performed, to enable negative test results to be distinguished from cases where the test site reaction did not occur because the sample did not in fact reach the test site, for example. This can give added confidence that the test was validly performed.

The control site could in some examples be disposed within a non-constricted portion of the fluid flow channel, such as in a subsequent portion of the fluid flow channel lying beyond the constricted portion. However in other examples the control site may also be disposed within the constricted portion of the fluid flow channel. This can be advantageous to ensure that both the test and the control site are exposed to a corresponding concentration of target analyte and substance so as to have an approximately equivalent limit of sensitivity.

The cross-sectional area of the constricted portion need not be constant throughout the length of the constricted portion. Similarly, the cross-sectional area may vary to some extent within the other portion. Hence, in general the maximum cross sectional area of the constricted portion can be smaller than the minimum cross-sectional area of the other portion of the fluid flow channel.

The cross-sectional area within the constricted portion may be reduced relative to the other portion in different ways. In one example, the cross-sectional area may be controlled by reducing the width of the fluid flow channel in a direction parallel to the plane of the substrate. Hence, a maximum width, c, of the constricted portion of the fluid flow channel in a direction parallel to the plane of the substrate may be narrower than a minimum width, w, of the other portion of the fluid flow channel in the direction parallel to the plane of the substrate. The ratio w/c may be chosen by the designer of the device depending on the desired trade-off between the limit of sensitivity and the total time taken for the test result to be observable. In general, the greater the ratio w/c, the greater the reduction in the limit of sensitivity. In some examples w/c may be greater than or equal to 1.2. More particularly, the ratio w/c may be: greater than or equal to 1.4; or greater than or equal to 1.6; or greater than or equal to 1.8; or greater than or equal to 2; or greater than or equal to 2.2; or greater than or equal to 2.4; or greater than or equal to 2.6; or greater than or equal to 2.8; or greater than or equal to 3; or greater than or equal to 3.2; or greater than or equal to 3.4; or greater than or equal to 3.6; or greater than or equal to 3.8; or greater than or equal to 4; or greater than or equal to 4.2; or greater than or equal to 4.4; or greater than or equal to 4.6; or greater than or equal to 4.8; or greater than or equal to 5; or greater than or equal to 5.2; or greater than or equal to 5.4; or greater than or equal to 5.6; or greater than or equal to 5.8; or greater than or equal to 6; or greater than or equal to 6.2; or greater than or equal to 6.4; or greater than or equal to 6.6; or greater than or equal to 6.8; or greater than or equal to 7; or greater than or equal to 7.2; or greater than or equal to 7.4; or greater than or equal to 7.6; or greater than or equal to 7.8; or greater than or equal to 8; or greater than or equal to 8.2; or greater than or equal to 8.4; or greater than or equal to 8.6; or greater than or equal to 8.8; or greater than or equal to 9; or greater than or equal to 10; or greater than or equal to 11; or greater than or equal to 12; or greater than or equal to 13; or greater than or equal to 14; or greater than or equal to 15; or greater than or equal to 16; or greater than or equal to 17; or greater than or equal to 18; or greater than or equal to 19; or greater than or equal to 20. For example, the constriction width c could range from 0.25 mm to 4 mm, and the total channel width in the other portion of the channel could range from 4 mm to 5 mm (of course other widths are also possible). The ratio w/c could be in the range 1.2 to 25, for example.

The difference w-c between the overall channel width and the width within the constricted portion may be greater than the manufacturing tolerance with which the wall position of the channels can be controlled. For example w-c may be greater than or equal to 0.8 mm. In other examples w-c may be: greater than or equal to 1 mm; or greater than or equal to 1.2 mm; or greater than or equal to 1.4 mm; or greater than or equal to 1.6 mm; or greater than or equal to 1.8 mm; or greater than or equal to 2 mm; or greater than or equal to 2.2 mm; or greater than or equal to 2.4 mm; or greater than or equal to 2.6 mm; or greater than or equal to 2.8 mm; or greater than or equal to 3.0 mm; or greater than or equal to 3.2 mm; 3.4 mm; or greater than or equal to 3.6 mm; or greater than or equal to 3.8 mm; or greater than or equal to 4 mm.

Another approach for constraining the cross-sectional area can be to reduce the thickness of the constricted portion of the fluid flow channel in a direction perpendicular to the plane of the substrate. For example this could be performed by forming a partial barrier of less porous material than the substrate material, with the partial barrier extending partially (but not completely) across the thickness of the substrate, and being provided at part of the substrate corresponding to the constricted portion, but not being provided (or being less thick) in the other portion of the fluid flow channel. The thickness of the channel may vary throughout the constricted portion and the other portion of the fluid flow channel. Hence, a maximum thickness of the constricted portion of the flow channel in the direction perpendicular to the plane of the substrate may be less than a minimum thickness of the other portion of the flow channel. Hence, within the constricted portion, the barrier concentrates the fluid into a smaller cross-sectional area to provide the effects described above. For example, the laser directed write technique discussed in more detail below can be used to form the partial barriers.

In other approaches the reduction in width and reduction in thickness of the fluid flow channel can be combined, to constrain the cross-sectional area of the constricted portion of the fluid flow channel in two dimensions simultaneously.

Regardless of whether the width or thickness of the constricted portion is reduced (or both are reduced), a length of the constricted portion along the fluid flow direction may be greater than or equal to 4 mm. For example, a distance between an entry region of the constricted portion (where the cross sectional area is narrowed compared to the cross-sectional area in an initial portion of the fluid flow channel) and an exit portion of the constricted portion where the cross-sectional area is expanded again, or the distance between the entry region and the end of the fluid flow channel if there is no subsequent portion of larger cross-sectional area, may be greater than 4 mm. By providing a length of at least 4 mm the constricted portion may have sufficient length to provide an appreciable effect on the concentration of target analyte and flow rates within the medium passing the test site, to achieve a limit of sensitivity reduction. Also this can provide sufficient space for forming a test site (and optionally a control site) which are large enough to register a visible result. In other examples the length of the constricted portion may be: greater than or equal to 4.2 mm; or greater than or equal to 4.4 mm; or greater than or equal to 4.6 mm; or greater than or equal to 4.8 mm; or greater than or equal to 5 mm; or greater than or equal to 5.2 mm; or greater than or equal to 5.4 mm; or greater than or equal to 5.6 mm; or greater than or equal to 5.8 mm; or greater than or equal to 6 mm.

Some LFDs, not falling within the scope of the invention, comprise a single fluid flow channel having a constricted portion and test site as discussed above.

According to the invention, single LFD comprises, within the same substrate, a plurality of fluid flow channels, where at least two of the fluid flow channels have a respective test site which comprises the same analyte detection substrate. Hence, the at least two flow channels are designed to perform the same type of test on the sample fluid (it will be appreciated that there could also be other channels on the same device performing other types of tests using a different analyte detection substrate). The at least two flow channels with the same type of analyte detection substrate have different channel boundary shapes. For example, in one embodiment the at least two fluid flow channels may include a first fluid flow channel having a constricted portion with a first cross-sectional area and a second fluid flow channel comprising a constricted portion with a second cross-sectional area different from the first cross-sectional area. Also the at least two fluid flow channels could include at least a first fluid flow channel having the constricted portion and a second fluid flow channel without the constricted portion so that the test site in the second fluid flow channel is in a region with a larger cross-sectional area than the cross-sectional area of the constricted portion of the first fluid flow channel. Either way, by providing multiple channels for applying the same test to the sample, but with different cross-sectional area in the portion comprising the test site, this can increase the overall dynamic range of the device as each channel has its calibration curve shifted to a different range of analyte concentrations, so that a test performed by the channels in combination may cover a larger range. That is, a channel with a larger constriction width or no constriction at all may be less easily saturated and so can indicate differences in colour intensity at higher analyte concentrations than a channel with a lower constriction width. Hence, by combining channels with wider or narrower cross-sectional areas in the portion comprising the test site, this could be used to enable a quantitative determination of the concentration of analyte (not just a qualitative indication of the presence of the analyte) over a wider range of analyte concentrations.

The channel boundaries of the constricted portion could be formed in different ways. For example, in one example the channel boundaries could simply comprise the edges of the substrate itself, but the substrate may be shaped to provide different channel widths or thicknesses throughout the length of the channel so as to apply a constriction. For example a substrate such as paper could be cut in the region comprising the test site so that the channel is narrowed. This can provide a simple way of controlling the channel's cross-sectional area.

Alternatively the channel boundaries in the constricted portion may comprise structures formed within the body of the substrate, where the structures are less porous than the material of the substrate. In this case, the overall substrate may have a more regular width or thickness so as to be simpler to use for the user and less likely to tear for example. However, by forming structures within the substrate to impede the passage of fluid through the channel boundaries, this reduces the cross sectional area within the constricted portion and provide the effects discussed above. For example these structures could be formed by a range of techniques, one of which is the laser directed write technique discussed below.

The substrate material could be any porous material. Examples of substrate material may include paper, nitrocellulose, and/or sintered materials such as sintered glass. Paper is a particularly useful example because of its low manufacturing cost.

In a corresponding way, a method of manufacturing a lateral flow diagnostic device (LFD) may comprise steps of forming channel boundaries defining a fluid flow channel within a substrate of porous material, where the fluid flow channel comprises a constricted portion with a smaller cross-sectional area than another portion of the channel; and forming a test site disposed at a predetermined location along the fluid flow channel, where the test site comprises an analyte detection substance and is formed within the constricted portion of the fluid flow channel.

The method could be performed in various ways. For example the channel boundaries could be formed by cutting or shaping the substrate material. The forming of the test site can be performed by impregnating part of the substrate with the analyte detection substrate.

In one example, the channel boundaries may be formed by applying radiation onto a radiation-sensitive substance impregnated in the substrate, to change the radiation-sensitive substance from a first state to a second state through at least part of the thickness of the substrate. This can provide a cost-effective and relatively simple to control approach for manufacturing the constrictions, which is more easily upscaled for mass production of LFDs at affordable costs.

In one example the radiation-sensitive substance may be deposited at selected locations on the substrate corresponding to the channel boundaries. By selectively depositing the substance only at the location of the channel boundaries this saves cost by reducing the amount of radiation-sensitive substance needed and reducing the need for subsequent development steps of the method to remove unconverted radiation-sensitive substance from portions of the substrate material other than the channel boundaries.

The radiation-sensitive substance could be one of a range of materials but in general it may be a substance which is at least one of: less permeable fluid more solid, or less soluble to a developer, when in the second state than in the first state. Examples of such materials are given below.

Another example may provide a lateral flow diagnostic device made by the method described above.

Specific examples of the techniques discussed above are provided below. It will be appreciated that the invention is not limited to these particular examples. The scope of the invention is defined and restricted by the appended claims.

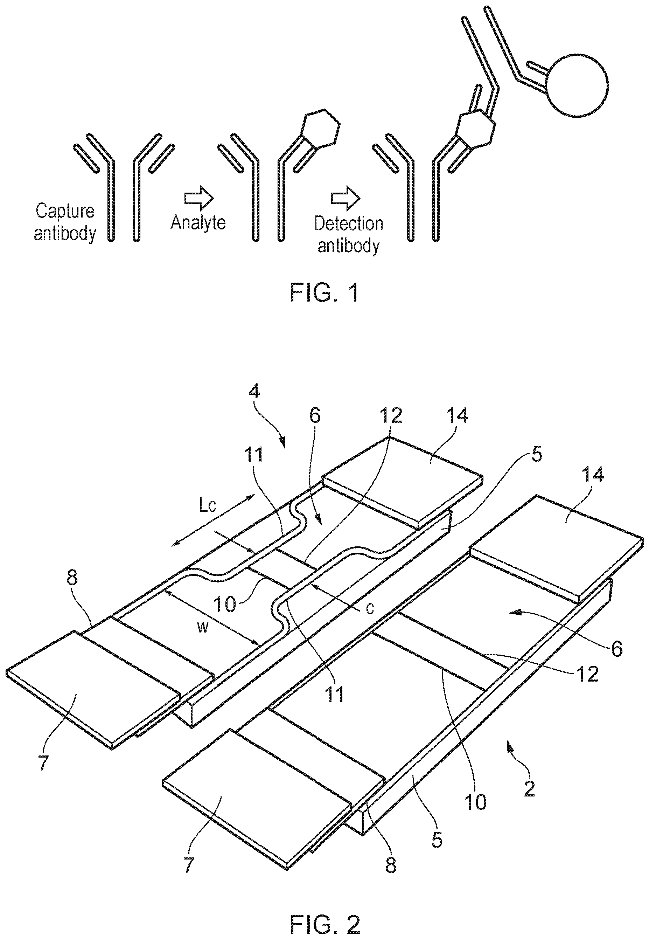

LFDs may be constructed using porous material including nitrocellulose or paper. LFDs can facilitate various tasks, one being the capture and detection of a target analyte. There is no need for external equipment as the sample fluids can be transported by the capillary action of these porous materials. LFDs implement two commonly used formats for lateral flow assays, namely the sandwich and the competitive assay. As seen in Figure 1, in the sandwich format the analyte in the sample binds to a labelled antibody, usually conjugated with gold nanoparticles, and the complex is transported along the strip until it encounters a capture antibody which is specific to the analyte and is immobilised on the substrate in the form of a line or spot forming the test site. The analyte-detection antibody complex is then visible as a coloured test line or spot that can be seen by eye or can be measured using a detector such as a scanner or a camera. After the test line there is also a control line which is used to clearly demonstrate that the assay is working properly and has immobilised antibodies that are specific to the conjugate antibody labelled with a nanoparticle. Capture of these conjugate antibodies at the control line again produces a colour change that verifies correct completion of the assay. Surplus reagents, buffers, and assay fluids are further transported toward an absorbent pad and are removed from the device as waste.

A schematic of a standard LFD 2 and our proposed constricted flow LFD 4 can be seen in Figure 2. Both LFDs 2, 4 comprise a substrate 5 of porous material (in this example nitrocellulose), on which is formed a fluid flow channel 6 along which sample fluid is passed when the device is used for a test. A sample pad 7 and conjugate pad 8 are provided at an inlet of the fluid flow channel 6. The sample pad 7 can be dipped into the sample fluid, so that the sample passes through the conjugate pad to bind to the conjugate substance, and flows through the fluid flow channel 6. A test site 10 and control site 12 are provided downstream from the sample pad 7 and the conjugate pad 8. The test site 10 and control site 12 each comprise a linear strip passing across the fluid flow channel with the line oriented perpendicular to the fluid flow direction. The test site 10 is impregnated with a test reagent (analyte detection substance) which reacts with the target analyte (or to the target analyte bound with the conjugate substance from the conjugate pad 8) to produce a visible colour change at the test site 10. The control site 12 is impregnated with a control substance which reacts with the conjugate substance or to other molecules of the sample fluid to provide a visible colour change at the control site 12, regardless of whether the target analyte is present. An absorbent pad 14 is provided at the end of the fluid flow channel 6 to absorb fluid which reaches the end of the channel.

In the standard LFD 2, the cross-sectional area of the fluid flow channel (i.e. the width of the channel multiplied by the thickness) is constant through its length (here the "length" refers to the dimension corresponding to the fluid flow direction). For example, the boundaries of the channel may simply be the edges of the substrate 5.

In contrast, in the constricted flow LFD 4, the test site 10 and control site 12 are formed within a constricted portion (marked L_{c}) of the fluid flow channel which has a smaller cross-sectional area than an initial portion of the channel 6 between the sample/conjugate pads 7, 8 and the constricted portion L_{c} and a subsequent portion of the channel 6 between the control site 12 and the absorbent pad 14. In this example, the width c of the constricted portion of the channel is narrower than a width w of the initial and subsequent portions of the channel 6. In this example, the constriction is produced by forming channel boundary structures 11 within the body of the substrate material, but an alternative way of constraining the channel width could be to cut the substrate so that the edges of the substrate become closer together in the portion comprising the test site 10 and control site 12.

Hence, spatial constrictions are used to influence the flow of the samples within the device. The constriction is shaped as follows: an ingress point gradually reduces the width of the flow path over a length of a few mm, followed by a length L_{c} of reduced-width flow path and finally an egress point where the flow path width returns to its original size, as seen in Figure 2.

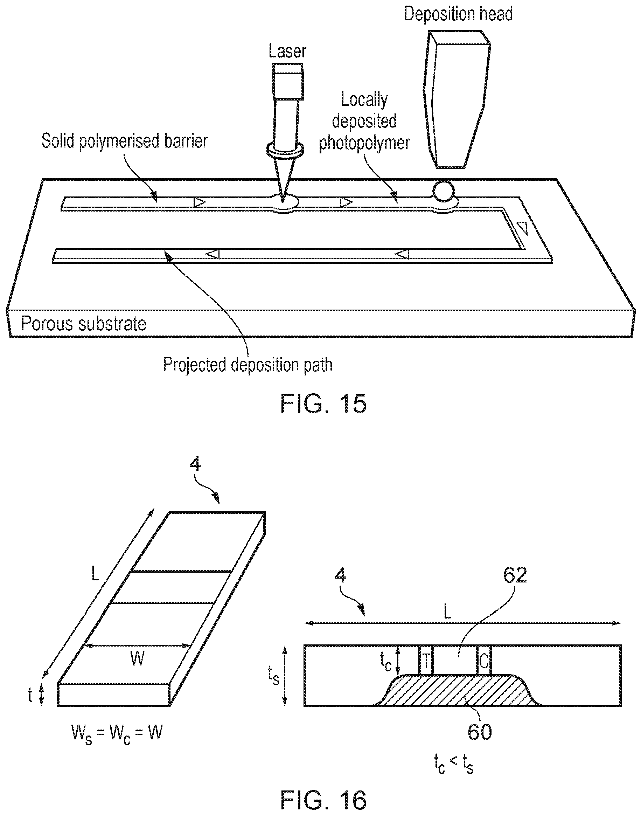

The LFDs can be made using a laser-directed write (LDW) manufacturing technique. LDW is a non-lithographic approach with high flexibility, which has no specialist equipment and material requirements and hence presents the potential to be up-scaled for mass-production of paper-based devices at affordable costs. In one example, the LDW technique comprises the immersion of the membrane in a photopolymer solution, a subsequent photopolymerisation with a laser beam, followed by the immersion of the membrane in a solvent to remove any unpolymerised residue. However, a decrease in the total number of fabrication-steps would however not only make this LDW process more efficient as a consequence of reduced fabrication times, but would also make it more cost-effective because of the reduced usage of expensive reagent, translating it into a truly mature technique appropriate for commercial manufacture. Hence, in another example this technique can be improved by including a deposition tool that allows localised (selective) deposition of the photopolymer only at specific locations on the paper where the fluid containing wall/structures need to be formed within the substrates to define the microfluidic device. This selective photopolymer deposition eliminates the (global) soaking step required to impregnate the photopolymer within the paper, prior to the laser illumination step, and furthermore also makes redundant the subsequent solvent developing step. An example LDW setup that allows the implementation of this improved methodology is illustrated in Figure 15. As shown in the schematic, the photopolymer is locally deposited onto the paper substrate with a deposition nozzle at locations pre-defined by the user's device design. A laser beam subsequently follows the deposition head and illuminates the deposited patterns thereby inducing photo-polymerisation of the polymer. The polymerised patterns define the fluidic walls that serve as demarcation barriers that confine and transport the liquids within the paper device.

To demonstrate the advantage of specifically designed flow-paths for an improved LFD performance, we have chosen to implement the detection of C-reactive protein (CRP). Of course other tests could also be implemented using such a LFD. CRP was chosen as the target analyte as it is well understood inflammation marker, heart attack marker and is also an excellent indicator for whether an infection is caused by a viral or a bacterial pathogen. CRP is normally present in very low concentrations in healthy individuals (<5 µg/ml) but rises significantly for unwell patients to values greater than 500 µg/ml. High concentrations of CRP in the bloodstream are not indicative of a specific disease, but quantitative measurements can provide valuable information for the diagnosis and treatment of a patient, and this is why CRP detection is broadly used in the primary care setting in conjunction with testing for other biomarkers.

For the purpose of measurements of the time a fluid takes to traverse through the devices, we made and tested LFDs with constriction widths c ranging from 1 mm to 5 mm in increments of 0.5 mm. For evaluating the implementation of the CRP assay only devices with constriction widths ranging from 1 mm to 5 mm with a 1 mm increment were used. These devices were made with the laser directed-write (LDW) approach discussed in more detail below. Of course, other techniques could also be used to produce the constricted flow LFDs.

The fluidic channels were fabricated within backed nitrocellulose membrane purchased from Sartorius (UniSart ^{®} CN95) and the absorbent pads were cellulose-based filter papers from GE Healthcare (CF1^{®})

The capture and detection antibodies used were from a DuoSet ^{®} ELISA development system (R&D Systems DY1707). The protein standard was recombinant human C-reactive protein expressed in E. Coli (Sigma Aldrich C1617). The control line antibody was an anti-mouse Goat IgG from R&D Systems (AF007). The streptavidin-conjugated gold nanoparticles were obtained from BBI solutions (BA.STP40). The BSA and PBS used in the solutions were obtained from Sigma Aldrich (A2058, P3813). The photopolymer used was Desolite 3471-3-14 from Desotech. The laser used for the LDW process was a 405 nm continuous wave diode laser (MLD^{™} 405 nm, Cobolt ^{®} AB) with a maximum output power of ~110 mW. The specialised polymer dispenser was a PICO Pµlse ^{®} jet valve on a 3-Axis EV series automated dispensing system purchased from Nordson. The reagent dispenser was a XYZ3210 platform with a Biojet HR ^{®} solenoid dispenser from Biodot.

The initial set of experiments involved studying the implications of the constricted flow designs on the flow time of the LFDs. Two sets of measurements were taken for devices with different constriction widths ranging from 5mm (a standard LFD strip width) to 1mm. All measurements were performed by adding 30 µl of red dye in microtiter wells and then immersing the inlet of the nitrocellulose membrane in the wells. Red dye was used instead of water for better visualisation of the flow and hence accuracy of timing.

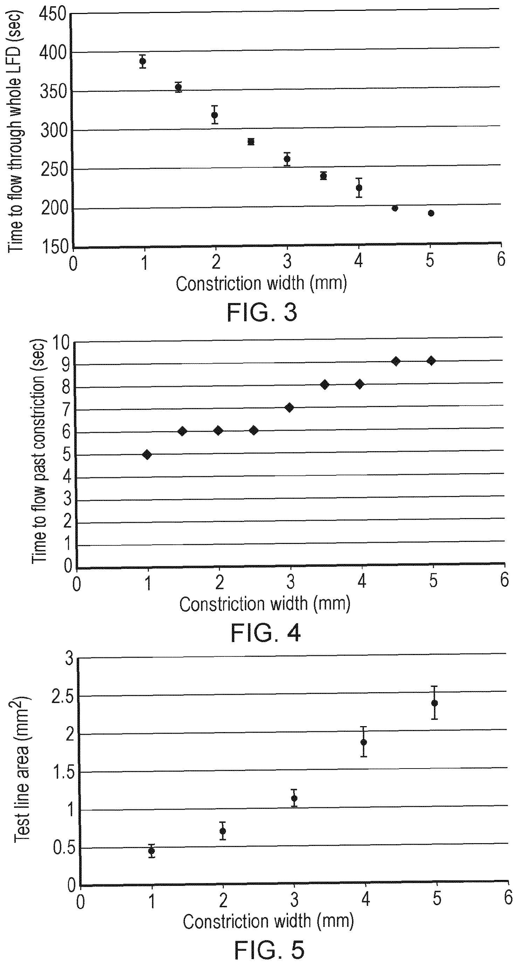

One measurement determined the time required for 30 µl of the red dye to be transported, after being completely soaked at the source pad 7, through the constriction L_{c} and until it is absorbed by the absorption pad 14. Figure 3 shows the flow time for a 30 µl sample to flow through the whole LFD and be absorbed by the absorbent pad 14. As can be seen in Figure 3, the smaller the constriction is, the narrower the flow path, and hence the larger is the flow time. This experiment includes the time for a dry membrane to become fully wetted (measuring the rate of saturation) and the time required for a standard sample volume to be completely absorbed by the device. This increase in the flow time allows for an increased interaction time between the sample and the capture antibody at the test line, therefore producing an enhanced detection signal.

Another experiment (results shown in Figure 4) measured the time required for a red dye to flow past the constriction. As seen in Figure 4, the flow times that were measured at the exit of the constrictions were slightly reduced for flow paths that were narrowest. This reduction does not however have a major influence because the total flow times across the entire LFD (as in Figure 3) is almost two orders of magnitude greater. The increased total flow times result from longer flow times after the constriction because the constriction works as a source that provides a reduced liquid volume the follow-on flow-rate is determined by the liquid provided by the source.

Additionally, the area of each test line was measured from individual images and it was observed that the test area is reduced when the flow path is constricted, as seen in Figure 5. Since the area of the test line is reduced, all of the sample is directed through this confined area, with an enhanced concentration of the capture antibodies. A larger number of analyte molecules will therefore be bound to this smaller area, producing an increased colour intensity, and thus an enhanced sensitivity and limit of detection.

Photopolymer lines forming the individual strips with different constriction widths (1, 2, 3, 4, 5 mm) were prepared by dispensing monomer with the Nordson dispenser and subsequently polymerising the material according to the LDW technique. The photopolymer dispensing was done at a speed of 40 mm/s and a frequency of 100 Hz thus ensuring that a continuous monomer line and hence impenetrable fluidic walls are formed within the nitrocellulose. The temperature of the dispensing head was set at 70° C to reduce the viscosity and facilitate the proper flow of the photopolymer through the dispenser. The test and control lines were subsequently produced by dispensing the capture antibody (mouse anti-human CRP in 1% BSA/PBS) and the control antibody (anti-mouse IgG in 1% BSA/PBS) with a Biodot dispenser at a concentration of 1 mg/ml. The test lines and control lines were dispensed on the nitrocellulose membrane in the middle of the constriction, at a 12 mm distance from the inlet of the device. The reagent droplets dispensed to produce the test and control lines had a volume of 20 nl and a separation of 250 µm, and this ensured the formation of a continuous test/control line. The nitrocellulose membrane was then attached to a standard LFD backing card, and this was followed by the attachment of the cellulose-based absorbent pad such that it overlapped the nitrocellulose membrane by 2 mm. The striped devices were left to dry at room temperature overnight and then the card was cut along the polymerised lines with a manual cutter to obtain individual LFDs.

The CRP-assay which was implemented via the LFDs has a sandwich format, and works as follows. The biotinylated anti-CRP conjugate antibodies attached with streptavidin-gold nanoparticle complexes first bind to the CRP analyte from the sample. The CRP analyte then binds to the anti-CRP capture antibodies (in the test line), thus producing a colour signal at the test line only when the analyte is present in the sample. The conjugate-labelled antibodies also bind to the anti-mouse antibodies in the control line, thus producing a coloured signal regardless of the presence or absence of the CRP analyte in the sample. 15 µl of the detection antibody (biotinylated mouse anti-human CRP) was added to a microtiter well at a concentration of 1.6 µg/ml along with 15 µl of streptavidin coupled with 40 nm gold nanoparticles at OD 1, and 10 µl of recombinant human CRP standard at various concentrations ranging from 2.5 µg/ml to 40 ng/ml in a 1% BSA/PBS solution. The gold-conjugated detection antibodies were premixed with the CRP standard in a microtiter well plate for several minutes, and this was done so that the complex achieved an equilibrium condition before it was introduced to the LFD. The LFD was immersed in the conjugate antibody - sample solution, and the device was then left to run for several minutes (the time ranged between 3 min to 6 min depending on the test zone constriction) until all of the solution was wicked through the membrane and the result was immediately documented using a standard scanner. All assays were performed in triplicate using different batches of the same analyte concentrations.

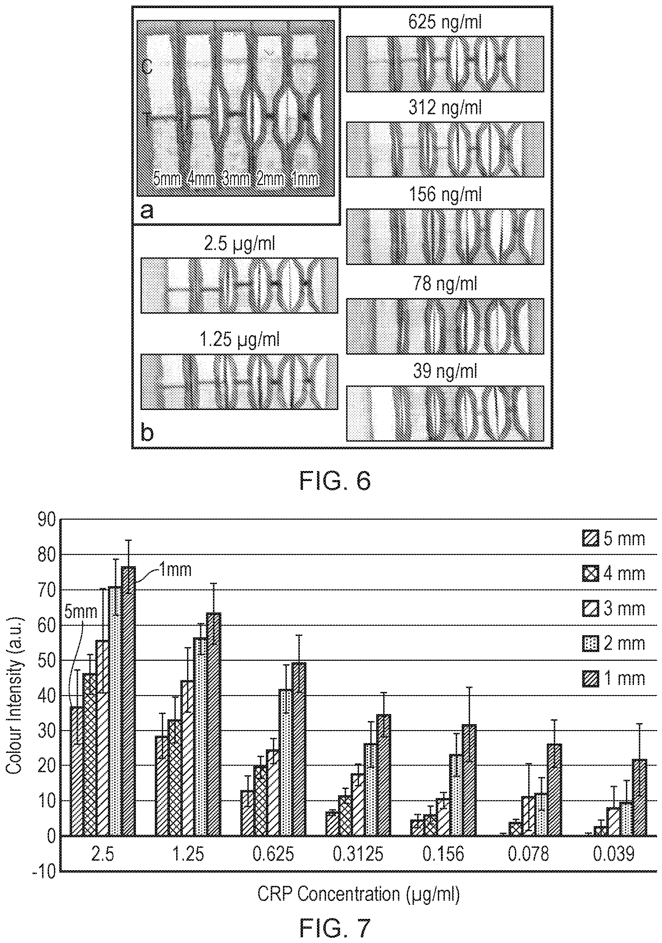

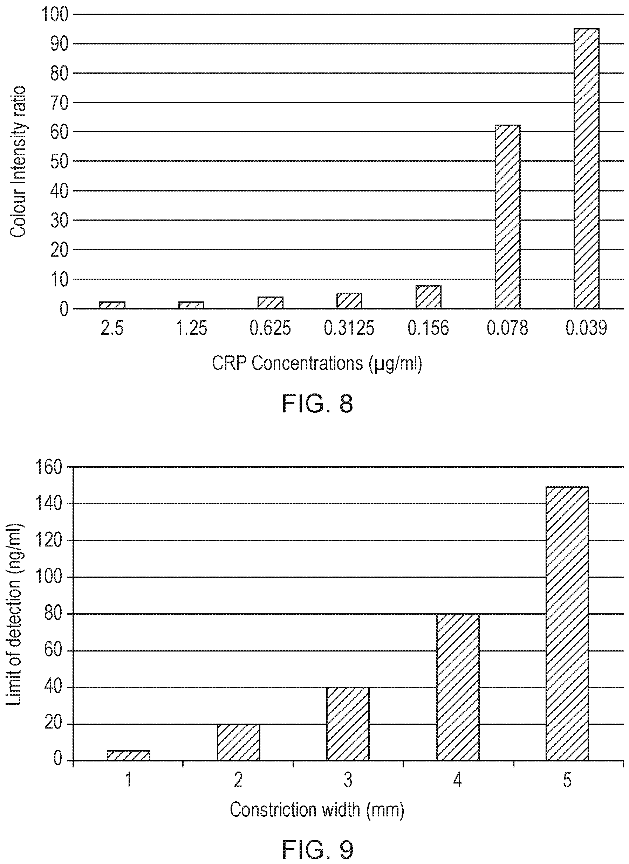

Figure 6 shows the scanned images of the CRP LFDs with different constrictions showing (a) the test and control lines and b) test lines of various CRP analyte concentrations. Note that in this example the control line C lies in the subsequent portion of the flow channel beyond the constriction. Following the experiments with varying constriction widths and analyte concentrations, the effect of the constrictions on the colour intensity produced at the test/control lines was evaluated. In order to quantify the results of the CRP assays and produce the colour intensity graphs, the photos were processed using the ImageJ software. The coloured test zone was selected and the mean grey values were extracted. These values were then and normalised by removing the background noise of each individual strip. The normalised colour intensity was then plotted against the channel width at the constriction, as can be seen in Figure 7, which illustrates the effect of the constriction widths on the colour intensity measurements for different concentrations of CRP. For each concentration, the bars arranged left to right correspond to constriction widths of 5 mm (left), 4 mm, 3 mm, 2 mm and 1 mm (right) respectively. For a CRP analyte concentration of 2.5 µg/ml the normalised colour intensity increased 2-fold for a decrease in the test zone width from 5 mm (the usual dimensions of a standard LFD) to 1 mm. It was observed that for smaller analyte concentrations, there was a substantial increase in colour intensity, for example, 5.3x for 312.5 ng/ml and 62x for 78 ng/ml. The increase in colour intensities for the different constriction widths becomes more apparent for reduced analyte concentrations. This significant change can be attributed to the fact that at high concentrations, both the standard 5 mm device and the LFDs with constrictions perform similarly but at lower concentrations the standard device cannot perform equally well due to its low limits of detection. The average ratio of the colour intensity of the 1 mm devices to that for the colour intensity of the 5 mm devices was calculated for the same range of analyte concentrations and it was found to be exponentially increasing towards smaller concentrations, as can be seen in Figure 8, which illustrates the ratio of colour intensities between the 1 mm and 5 mm constriction devices for a range of analyte concentrations.

Achieving a decrease of the limit of detection for LFDs has been an objective that has seen considerable research effort, but yet, LFDs have not found to be as sensitive as other standard laboratory techniques, and this has been one of the primary reasons that has hindered their widespread adoption for clinical diagnostic purposes. From the latter experiment, it can be inferred that the limit of detection can be greatly enhanced using constricted flow paths. Figure 9 illustrates the limit of detection for the CRP lateral flow assay depending on the constriction width on the membrane. As can be seen in Figure 9, the limit of detection of the CRP assay on a standard5 mm wide LFD was -160 ng/ml. However, on decreasing the width of the constrictions, the limit of detection is also decreased considerably, and for the case of the device with a 1 mm wide constriction, the limit of detection was found to be ~5 ng/ml, a 32-fold enhancement when compared to the standard LFD.

Hence, we have presented a novel strategy for improving the sensitivity and limit of detection of LFDs by fabricating spatial constrictions in the flow path of the nitrocellulose membranes within these devices. The enhanced signal that is produced within these constriction-enabled LFDs is, as per our understanding because of two reasons - firstly, the test zone is decreased in area which means that the AuNP-conjugated antibodies and the sample is therefore concentrated, and secondly, the overall flow rate is decreased, therefore allowing for longer interaction time between the capture antibody and the sample. This therefore allows for the fabrication of LFDs with enhanced sensitivity as they can produce increased colour intensity for the same analyte concentration. We have evaluated different designs and depending on the analyte concentration, these designs exhibited a significant improvement of the observed signal. Additionally, the limit of detection of such a device was found to be lowered considerably, allowing for use of such devices with samples with very low analyte concentrations that would otherwise be impossible to detect. Sensitivity and limit of detection are the two key parameters that LFDs have been inadequate at, and by using a uniquely designed flow path created with a simple laser manufacturing technique we believe we can enable the drive for the use of LFDs as efficient, standard laboratory diagnostic tools.

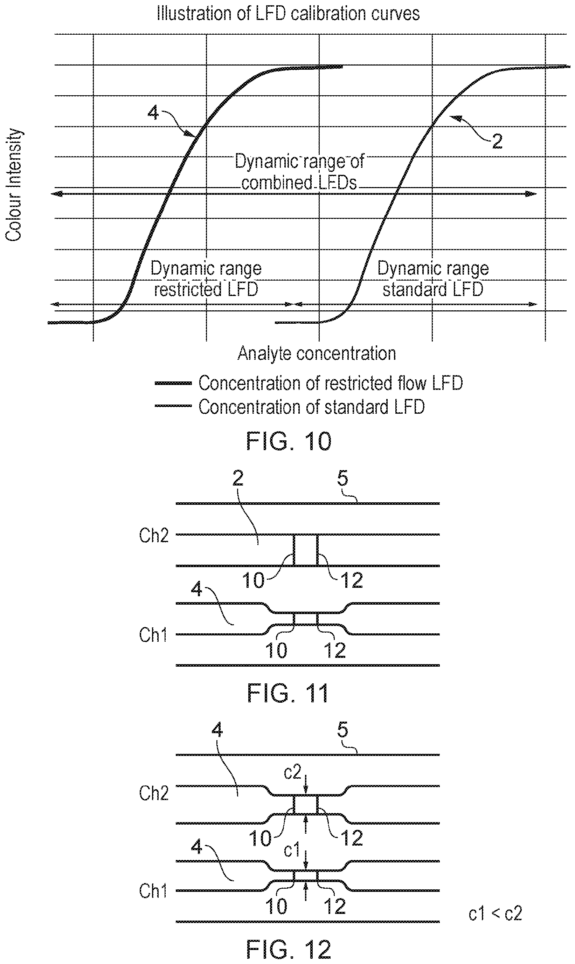

Figure 10 illustrates calibration curves showing variation in colour intensity of the test site 10 following the test reaction, for different analyte concentrations, for the standard LFD 2 and constricted flow LFD 4 respectively. As shown in Figure 10, the constricted flow LFD 4 essentially shifts its calibration curve towards lower concentrations, as it has a lower limit of detection and saturates at lower concentrations. The standard LFD 2 (without any constricted portion in the flow channel 6) of the same CRP assay will have its curve towards the higher concentrations as its detection limit is higher and saturates at higher concentrations.

Hence, as illustrated in Figure 11, a standard LFD 2 and constricted flow LFD 4 can be combined as parallel channels within the same substrate to form a single device. Both the standard and constricted flow channels have a test site 10 with the same analyte detection substance (so rather than implementing tests for different analytes, they provide parallel instances of the same test). However, the test site 10 of the constricted flow LFD 4 is within a constricted portion with a reduced cross-sectional area compared to other parts of the channel, while in the standard LFD 2 the channel cross-sectional area is constant throughout the channel and larger than the cross-sectional area of the constricted flow LFD 4. Hence, by combining standard and constricted flow LFDs within a single device, we can effectively combine their dynamic ranges and therefore detect analyte concentrations from very low to very high concentrations. That is, the overall device has a wider dynamic range of concentrations over which a quantitative estimate of the analyte concentration can be made based on the detected colour intensity of the test site 10 in each of the channels 2, 4. This cannot be implemented in such a simple and easy way using other techniques to enhance the sensitivity and dynamic range of the device.

Figure 12 shows an embodiment in accordance with the invention of increasing the dynamic range of analyte concentration detection. In this example, two constricted flow LFD channels 4 are provided in the same substrate 5. The first constricted flow channel CL1 has a constricted portion with a smaller channel width c1 (and hence smaller cross-sectional area) than the width c2 (and cross-sectional area) of the constricted portion of the second constricted flow channel CL2. Both channels CL1, CL2 provide a test site 10 impregnated with the same analyte detection substance. Hence, this provides a similar effect to that of Figure 11, to increase the dynamic range of the combined device as shown in Figure 10.

It will be appreciated that other examples may provide more than two channels with different cross-sectional areas in the portion of the channel comprising the test site 10. Also, further channels could also be provided with test sites comprising different analyte detection substances, in addition to the multiple channels having the same analyte detection substance - in this case the additional channels with a different analyte detection substance would provide different a test functionality rather than increasing the dynamic range of detection.

As discussed above, one way of forming the constricted portion can be to cut or shape the sides of the substrate to provide a narrower substrate width in the portion corresponding to the constricted portion of the fluid flow channel.

However, another approach can be to retain the rectangular shape of the substrate, but to form channel boundary structures 11 within the body of the substrate to impede flow of fluid and hence define a narrower channel. An approach for forming these structures using a laser-directed write (LDW) technique will now be described in more detail. The LDW approach may be more easily scalable for mass product at reasonable cost. In this description, "radiation" refers to any form of radiative energy, including energy transferred by waves or particles. Examples include electromagnetic radiation (including any part of the electromagnetic spectrum, e.g. radiofrequency radiation, microwaves, visible light, infrared radiation, ultraviolet radiation, X-ray radiation, gamma radiation etc.); radiation of particles (e.g. electron beam, ion beam, etc.), or acoustic radiation (e.g. ultrasound). "Radiation-sensitive substance" refers to any substance or combination of multiple substances which, when radiation is applied thereto, changes from a first state to a second state. In some embodiments, the substance is less permeable, more solid, and/or less soluble to a developer in one of the first state and the second state than the other. In some embodiments, the radiation-sensitive substance comprises one or more polymerisable substances, as described herein. "Depositing" refers to depositing of the radiation-sensitive substance on the substrate. "The application of radiation" refers to the application of radiation to at least part of the substrate, such that at least part of the substrate is exposed to radiation.

The following description describes a technique suitable for defining fluid-containing regions such as flow channels, wells and reservoirs in a substrate or membrane made from porous material, such as paper, nitrocellulose or sintered glass. The regions are defined by barriers or walls within the substrate at the boundaries of each region, such as at each side of a channel. The walls are formed from a less permeable state of a radiation-sensitive substance, for which a state change is induced by exposure to radiation energy. Hence, radiation is used to create regions of less permeable material (e.g. the walls or barriers of fluid-containing regions) within areas of porous material (e.g. the inside of the channels, reservoirs and similar, plus areas outside the fluid-containing regions) in a porous substrate such as paper or nitrocellulose. To achieve this, a radiation-sensitive substance is used which can be altered or changed from a first state to a second state by the application of radiation. One of the first state and the second state is a less permeable, more solid or less soluble state. In some cases that one of the first/second states may be an impermeable state, in which the substance takes the form of a material that impedes flow of fluid and can therefore be used to create a physical wall or barrier to fluid flow within the material of the substrate. The less permeable state may in some examples be hydrophobic, as this helps in containing larger volumes of aqueous fluids within the flow channel structure for longer times. However, radiation-sensitive substances having a less permeable state which is not hydrophobic can also be used, and may provide adequate fluid containment. The other more permeable state is typically a liquid state so that the radiation-sensitive substance can be conveniently applied to the substrate in a pattern corresponding to the desired locations of the walls or barriers. For example, the substance is deposited onto the substrate surface in the required pattern and then permeates or soaks into the substrate below the pattern so that a volume of material extending through the thickness of the substrate becomes impregnated with the radiation sensitive substance.

Any radiation-sensitive substance, compound, chemical or material which behaves in this way and which can be impregnated into the substrate material (either impregnated throughout the substrate, or selectively in a desired pattern) can be employed in the LDW technique.

In some examples, the radiation-sensitive substance is transformed by radiation exposure from a first state to a second state which is less permeable, more solid and/or less soluble to developer, and hence can be considered to operate in a negative regime. In the case of a less permeable state, it may be a partially permeable state or a fully impermeable state. In some examples, the less permeable state may also be seen as a solid state or a state in which the substance is insoluble to a developer.

In other examples, the radiation-sensitive substance may operate in an opposite, positive regime, in which exposure to radiation transforms the substance from a first state to a second state which is more permeable to fluid, less solid and/or more soluble to developer. With this approach, the substance can be laid down in areas of the substrate corresponding to both the walls/barriers and the fluid containing regions within the walls/barriers. Radiation may then be applied locally to the fluid-containing regions but not the walls/barriers to selectively convert the fluid containing regions to the more permeable, less solid or more soluble state (e.g. a liquid state). A subsequent development step may then use solvent to remove the radiation-sensitive substance that is in the first state, but not the walls/barriers corresponding to substance in the second state.

Use of a negative-type radiation-sensitive substance may often be more convenient (it may require less radiation-sensitive substance to be deposited and less removal of excess radiation-sensitive substance using a solvent or other developer), and for the subsequent description a negative-type substance will be described. However, it will be appreciated that positive-type substances could also be used.

For a negative-type radiation-sensitive substance, exposure of the substance to radiation forms less permeable, more solid and/or less soluble material within the substrate in the exposed areas. Hence, if the radiation is applied in the form of a beam which is moved to follow lines of the deposited pattern, the substance is hardened along the lines so as to become walls or barriers for fluid-containing regions. The width of the beam can be chosen relative to the width of lines in the pattern to be narrower, the same or wider, and aligned exactly or with an overlap, so as to expose all or only some of the substance. Various effects can thereby be achieved; these are described in more detail later.

Radiation-sensitive substances suitable for use in the LDW technique include materials sometimes referred to as polymerisable substances, photoresists, and radiation-curable resins and adhesives, inks and other similar materials.

Typically, the polymerisable substance is a substance containing molecules (monomers) which, on the application of radiation, bond to one another to form a polymer. The polymer may be more permeable or less permeable than the polymerisable substances from which it is formed. Typically, the polymer is less permeable than the polymerisable substances from which it is formed. In some examples, the more permeable state may be a liquid state and the less permeable state may be a state which is more solid, firm or hard. In some examples, the polymer may be more or less soluble to a developer than the polymerisable substances from which it is formed.

The polymerisable substance may comprise (or consist of) a monomer molecule. In this specification the term "monomer molecule" means a molecule capable of undergoing polymerisation to thereby form the constitutional units of a polymer.

The polymer formed from the monomer molecules is typically an organic polymer. A large number of organic polymers are known in the art. Examples of particular classes of organic polymers suitable for use include polyolefins, polyesters, polycarbonates, polyamides, polyimides, polyether sulfones, and mixtures or derivatives thereof.

In the LDW technique, the monomer molecule is typically capable of radiation-initiated polymerisation (i.e. polymerisation initiated by the application of radiation, as defined herein). Examples of such monomer molecules include ethylenically unsaturated monomers. Any compound having a carbon-carbon double bond and which is capable of being polymerised by the application of radiation may function as an ethylenically unsaturated monomer.

In one embodiment, the ethylenically unsaturated monomer may be an olefin: in other words, an unsubstituted, unsaturated hydrocarbon (such as ethylene, propylene, 1-butene, 1-hexene, 4-methyl-1-pentene or styrene). In this specification polymers formed by polymerising such monomers are termed 'polyolefins'.

In another embodiment, the ethylenically unsaturated monomer is an ethylenically unsaturated hydrocarbon substituted with one or more functional groups; examples of such functional groups include the substituents defined and exemplified below in relation to the substituent group R₂ on an acrylate or methacrylate group; further examples include halogen atoms, particularly fluorine atoms (examples of olefins substituted with such groups include vinylidene fluoride or tetrafluoroethylene) or chlorine atoms (examples of olefins substituted with such groups include vinyl chloride and vinylidene dichloride), carboxylic acid or carboxylic ester groups (examples of olefins substituted with such groups include acrylic or methacrylic monomers, as described and exemplified below), nitrile groups (examples of olefins substituted with such groups include acrylonitrile and methacrylonitrile). In this specification polymers formed by polymerising such monomers are termed 'substituted polyolefins'.

In one embodiment, the ethylenically unsaturated monomer is a (meth)acrylate monomer. These are monomers of the formula: 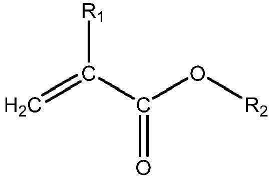 wherein R₁ is hydrogen or methyl, and R₂ is hydrogen or a substituent, or two groups R₂ together form a linker group. When R₁ is hydrogen, the monomer is an acrylate monomer. When R₁ is methyl, the monomer is a methacrylate monomer.

When R₂ is a substituent, the substituent may comprise or consist of a hydrocarbyl group, typical examples of which include alkyl, alkenyl, alkynyl, cycloalkyl, heterocyclyl, aryl and heteroaryl groups. In one embodiment the substituent may comprise or consist of an alkyl group. In this specification the term "alkyl group" means a saturated, monovalent, hydrocarbon moiety. The alkyl group is typically a C₁₋₃₀ alkyl group, such as a C₁₋₁₀ alkyl group, such as a C₁₋₆ alkyl group, such as a C₁₋₄ alkyl group, such as a methyl, ethyl, n-propyl, isopropyl, n-butyl, isobutyl, sec-butyl, or tert-butyl. The alkyl group may be substituted with one or more (typically only one) substituent, examples of which include halogen (especially fluorine or chlorine), hydroxy, nitrile (-CN), carboxylic acid (-CO₂H) and carboxylic ester (-CO₂R') where R' is hydrogen or a substituent, typically a C₁₋₆ alkyl group or a benzyl group.

In one embodiment the substituent may comprise or consist of an alkenyl group. In this specification the term "alkenyl group" means a monovalent, hydrocarbon moiety having at least one carbon-carbon double bond. The alkenyl group is typically a C₂₋₁₀ alkenyl group, such as a C₂₋₆ alkenyl group. The alkenyl group may be substituted with one or more (typically only one) substituent, examples of which include halogen (especially fluorine or chlorine), hydroxy, nitrile (-CN), carboxylic acid (-CO₂H) and carboxylic ester (-CO₂R') where R' is hydrogen or a substituent, typically a C₁₋₆ alkyl group or a benzyl group.

In one embodiment the substituent may comprise or consist of an alkynyl group. In this specification the term "alkynyl group" means a monovalent, hydrocarbon moiety having at least one carbon-carbon triple bond. The alkynyl group is typically a C₂₋₁₀ alkynyl group, such as a C₂₋₆ alkynyl group. The alkenyl group may be substituted with one or more (typically only one) substituent, examples of which include halogen (especially fluorine or chlorine), hydroxy, nitrile (-CN), carboxylic acid (-CO₂H) and carboxylic ester (-CO₂R') where R' is hydrogen or a substituent, typically a C₁₋₆ alkyl group or a benzyl group.

In one embodiment the substituent comprises or consists of a cycloalkyl group. In this specification the term "cycloalkyl group" means a monovalent, saturated, cyclic hydrocarbon group. The cycloalkyl group is typically a C₃₋₁₀ cycloalkyl group, such as a C₃₋₈ cycloalkyl group, such as a C₄₋₆ cycloalkyl group. The cycloalkyl group may be substituted with one or more (typically only one) substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group.

In one embodiment the substituent comprises or consists of a heterocyclyl group. In this specification the term "heterocyclyl group" means a monovalent, saturated, cyclic group, having 1 to 4 heteroatoms selected from nitrogen, oxygen and sulphur. The heterocyclyl group is typically a 5- or 6-membered heteroaryl group, such as a tetrahydrofuryl, pyrrolidinyl, tetrahydrothienyl, oxazolidinyl, isoxazolidinyl, thiazolidinyl, isothiazolidinyl, thiadiazolidnyl, piperidinyl, piperazinyl or morpholinyl group. The heterocyclyl group may be substituted with one or more substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group.

In one embodiment the substituent comprises or consists of an aryl group. In this specification the term "aryl group" means a monovalent, unsaturated, aromatic group (i.e. an unsaturated group having 4n+2 pi electrons, where n is an integer, preferably 1 or 2). The aryl group is typically a C₆₋₁₀ aryl group, such as a phenyl or naphthyl group. The aryl group may be substituted with one or more substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group.

In one embodiment the substituent comprises or consists of a heteroaryl group. In this specification the term "heteroaryl group" means a monovalent, unsaturated, aromatic group, having 1 to 4 heteroatoms selected from nitrogen, oxygen and sulphur. The heteroaryl group is typically a 5- or 6-membered heteroaryl group, such as a furyl, pyrrolyl, thienyl, oxazolyl, isoxazolyl, thiazolyl, isothiazolyl, triazolyl, thiadiazolyl, tetrazolyl, pyridiyl, pyrimidyl, pyrazinyl or triazinyl group. The heteroaryl group may be substituted with one or more substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group.

Examples of acrylate and methacrylate monomers include acrylic acid (R₁ and R₂ are H) methacrylic acid (R₁ is methyl and R₂ is H), and acrylic and methacrylic esters such as methyl acrylate (R₁ is H and R₂ is methyl), ethyl acrylate (R₁ is H and R₂ is ethyl), 2-ethylhexyl acrylate (R₁ is H and R₂ is 2-ethylhexyl), hydroxyethyl methacrylate (R₁ is H and R₂ is 2-hydroxyethyl), butyl acrylate (R₁ is H and R₂ is butyl) and butyl methacrylate (R₁ is methyl and R₂ is butyl).

When two groups R₂ together form a linker group, the monomer is a diacrylate or dimethacrylate. The linker group may be an aliphatic chain (for example an alkylene group or an oxyalkylene group), an alicyclic linker ring (for example a cycloalkylene, arylene or heteroarylene ring), or a combination thereof.

In one embodiment the linker group comprises or consists of an alkylene group. In this specification the term "alkylene group" when used to define the linker group means an aliphatic, saturated, divalent, hydrocarbon moiety. The alkylene group is typically a C₁₋₃₀ alkylene group, such as a C₁₋₁₀ alkylene group, such as a C₁₋₆ alkylene group, such as a C₁₋₄ alkylene group, such as a methylene, ethylene, methylmethylene, propylene or butylene group, and especially an ethylene group. The alkylene group may be substituted with one or more (typically only one) substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group. In one embodiment, the substituent on the alkylene group links the alkylene group to the rest of the linker group, such as those defined and exemplified below.

In one embodiment the linker group comprises or consists of a cycloalkylene group. In this specification the term "cycloalkylene group" when used to define the linker group means a divalent, saturated hydrocarbon group. The cycloalkylene group is typically a C₃₋₁₀ cycloalkylene group, such as a C₃₋₈ cycloalkylene group, such as a C₄₋₆ cycloalkylene group. The cycloalkylene group may be substituted with one or more (typically only one) substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group. In one embodiment, the substituent on the cycloalkylene group links the cycloalkylene group to the rest of the linker group, such as those defined and exemplified below.

In one embodiment the linker group comprises or consists of an arylene group. In this specification the term "arylene group" when used to define the linker group means a divalent, unsaturated, aromatic group. The arylene group is typically a C₆₋₁₀ arylene group, such as a phenylene group or naphthylene group. The arylene group may be substituted with one or more substituent, examples of which include halogen (especially fluorine or chlorine), hydroxy, nitrile (-CN), carboxylic acid (-CO₂H) and carboxylic ester (-CO₂R) where R is hydrogen or a substituent, typically a C₁₋₆ alkyl group or a benzyl group. In one embodiment, the substituent on the arylene group links the arylene group to the rest of the linker group, such as those defined and exemplified below.

In another embodiment the linker comprises or consists of an oxyalkylene or polyoxyalkylene group. An oxyalkylene group has the formula:

-[CH(R₁)-CH(R₂)-O]-ₙ

wherein R₁ and R₂ are hydrogen or a C₁₋₄ alkyl group, such as a methyl group, and n is typically 1 to 350, such as 1 to 100, such as 1 to 50, such as 1 to 20, such as 1, 2, 3, 4, 5, 6, 7, 8, 9, or 10. When n is 1, the linker comprises an oxyalkylene group: when n is 2 or more, the linker comprises a polyoxyalkylene group. Typically the linker group is a oxyethylene or polyoxyethylene group (i.e. wherein R₁ and R₂ are hydrogen).

In another embodiment the linker comprises or consists of an ester (-C(=O)-O-) group. In another embodiment the linker comprises or consists of an amide (-C(=O)-N(R")-) group, where R" is hydrogen or a substituent, typically a C₁₋₆ alkyl group or a benzyl group. In another embodiment the linker comprises or consists of an ether (-O-) group.

In one embodiment, the linker comprises or consists of a urethane (-O-C(=O)-NR"-) group (where R" is as defined above).

In one embodiment, the linker group comprises both an alkylene group (as defined and exemplified above) and an oxyalkylene or polyoxyalkylene group (as defined above). The linker group may comprise an oxyalkylene or polyoxyalkylene group having two alkylene termini. In this embodiment, the oxyalkylene or polyoxyalkylene group may be bonded directly to the two alkylene termini or may be bonded via a linker group, typically an ester group.

In one embodiment, the linker group comprises both an alkylene group, cycloalkylene group and/or an arylene group (as defined and exemplified above) and one or more urethane groups (as defined above). In one embodiment, the linker group may be an alkylene, cycloalkylene group /or an arylene group having two urethane termini. In this embodiment, the alkylene, cycloalkylene group /or an arylene group may be bonded directly to the two urethane termini or may be bonded via a further linker group, such as those defined and exemplified above.

Examples of such diacrylates and dimethacrylates include alkylene diacrylate or dimethacrylates (where two groups R₂ together form alkylene, as defined and exemplified above, especially ethylene glycol diacrylate or dimethacrylate) and glycol ether diacrylates and dimethacrylates, such as polyalkylene glycol diacrylates and polyalkylene glycol dimethacrylates, where two groups R₂ together form an oxyalkylene or polyoxyalkylene group, as defined and exemplified above) polyethylene glycol dimethacrylate. The polyethylene glycol moiety of polyethylene glycol diacrylates and polyethylene glycol dimethacrylates typically has an average molecular weight ranging from 200 to 20,000, typically 200 to 1000.

Further examples of such diacrylates and dimethacrylates include urethane diacrylates or dimethacrylates (where two groups R₂ together form a linker including a urethane linkage, as defined and exemplified above). A particular example is the urethane di(meth)acrylate sold as OP-66-LS by DYMAX Corporation.

Further examples of acrylates include the acrylate monomer sold as ABELUX A4061T by DYMAX Corporation.

In another embodiment, the monomer is a mercapto ester. As is known to the person skilled in the art, mercapto esters have the formula R-C(=O)-SR' wherein R and R' are substituents, as defined above in relation to the substituents R₂ on an acrylate or methacrylate group, especially, alkyl, aryl or heteroaryl groups. These may be copolymerised with a number of other co-monomers, such as triallyl isocyanurate (CAS No. 1025-15-6) or tetrahydro-2-furanylmethyl methacrylate. Examples of co-monomer mixtures include those sold as Norland 61 and Norland 68 by Norland Products Incorporated.

The polymer formed from the monomers may be cross-linked. Typically, a cross-link is a region in the polymer from which at least four chains emanate, and is typically formed by reactions involving sites or groups on the existing polymer structure or by interactions between existing polymers. The region may be a direct bond between the polymer chains, a single atom (such as an oxygen or sulphur atom), a group of atoms (such as an alkylene group or alkyleneoxy group, as defined and exemplified above), or a number of branch points connected by bonds, groups of atoms, or oligomeric chains.

Cross-linking of the polymer chains can result in a network polymer. The degree of cross-linking of a network polymer may vary depending on the nature of the polymer and the conditions and reagents used to produce it. Examples of suitable reagents and conditions are well known to those skilled in the art. The degree of cross-linking can influence the mechanical strength of the polymer and the degree of permeability to a fluid.

The polymerisable substance may be polymerised by any method known to those skilled in the art. Examples of polymerisation methods include radical polymerisation (in which the reactive species which carry the polymerisation chain reaction are free radicals), cationic polymerisation (in which the reactive species which carry the polymerisation chain reaction are cations), anionic polymerisation (in which the reactive species which carry the polymerisation chain reaction are anions), or any combination thereof. It is preferred that the polymerisation method is radical polymerisation, as this mechanism is most easily induced by radiation.

In one embodiment, the monomer is polymerised in the presence of a photoinitiator. A photoinitiator is a chemical compound that decomposes into free radicals when radiation is applied. The photoinitiator may be a Type I or Type II photoinitiator. Type I photoinitiators undergo cleavage upon irradiation to generate two free radicals in which only one is reactive and proceeds to initiate polymerization. Type II photoinitiators form an excited state (e.g. a triplet state) upon irradiation but must abstract an atom or electron from a donor synergist, which then acts as the initiator for polymerization.

Examples of photoinitiators are well known to those skilled in the art. Examples of Type I photoinitiators include azobis(isobutyronitrile) (AIBN), peroxides such as benzoyl peroxide, benzoin ethers, benzil ketals, α-dialkoxyacetophenones, α-aminoalkylphenones, α-hydroxyacetophenones, and acyl phosphine oxides. Examples of Type II photoinitiators include diaryl ketones (benzophenones) such as benzophenone and substituted benzophenones, thioxanthones such as isopropyl thioxanthone and 2,4-diethylthioxanthone, and quinones such as benzoquinone, camphorquinone and anthraquinone.

In one embodiment, the radiation sensitive material comprises (or consists of) a photoresist. Photoresists are classified into two groups: positive resists and negative resists. In the context of the present technique, the term "positive resist" means a type of photoresist in which the portion of the photoresist that is exposed to radiation becomes more soluble to the developer (e.g. a solvent). The portion of the positive photoresist that is unexposed remains less soluble. For example, exposure to the radiation changes the chemical structure of the resist so that it becomes more soluble in the developer, and the exposed resist can be washed away by the developer solution, leaving windows of bare underlying material.

In contrast, in the context of the present technique, the term "negative resist" means a type of photoresist in which the portion of the photoresist that is exposed to radiation becomes less soluble to a developer. For example, when exposed to radiation, the negative resist becomes crosslinked/polymerised and more difficult to dissolve in the developer. The negative resist remains in the regions where it is exposed to radiation, and the developer solution removes the unexposed areas (although masks are not essential for the present technique, the term "negative" is derived from the fact that a mask used for negative resists contains the inverse or photographic 'negative' of the pattern to be transferred). In one embodiment, the photoresist is a negative photoresist.

The LDW technique is not limited to any particular radiation-sensitive substance. Radiation-sensitive or photosensitive materials other than those described above but which nevertheless behave in a similar manner may be used to implement the various embodiments of the LDW technique. The type of radiation (e.g. wavelength of electromagnetic radiation) and the level of energy density needed will depend on the choice of radiation-sensitive substance and the thickness and structure of the substrate. Various radiation-sensitive substances may require more than one form of radiation exposure, e.g. a heat treatment after a light exposure stage to harden or produce the required properties; methods according to various embodiments of the LDW technique may include such a step if necessary.

As examples of radiation-sensitive substances, the inventors have used the polymerisable substances DeSolite (registered trade mark) 3471-3-14 (from DSM Desotech Inc. or Chemtrec International, USA), in which the monomer is a glycol ether acrylate, and SUBSTANCE G (from MakerJuice, USA), in which the monomer is an acrylate ester, to implement embodiments of the LDW technique. As mentioned, however, other radiation-sensitive substances with the appropriate characteristics could be used.

In some examples, two or more different types of radiation-sensitive substances may be deposited on the substrate. For example, a first type of radiation-sensitive substance may be deposited in a first pattern and a second type of radiation-sensitive substance may be deposited in a second pattern. The different types of radiation-sensitive substance may have different properties, e.g. different permeability, different energy density (energy per unit area) of radiation required for converting the state of the substance, etc. This can allow for further control of the generation of substances in the fluid flow network, e.g. with a single source of radiation having a set energy density, structures of differing permeability can be created using the two or more different types of radiation-sensitive substance.

In the LDW method, a light-writing technique is used to create fluid flow channels by creating walls extending through the full thickness of a paper substrate. In one example, the substrate is completely impregnated with a light-sensitive substance by soaking the substrate in the substance, and a light beam is moved over the substrate to selectively harden the substance in the exposed areas only. After exposure, the substrate is subjected to a development stage in which a solvent is used to remove non-hardened light-sensitive substance which has not been exposed to the light. Hence, it is the relative motion between the light beam and the substrate which defines the pattern of solid material and hence the channel structure.

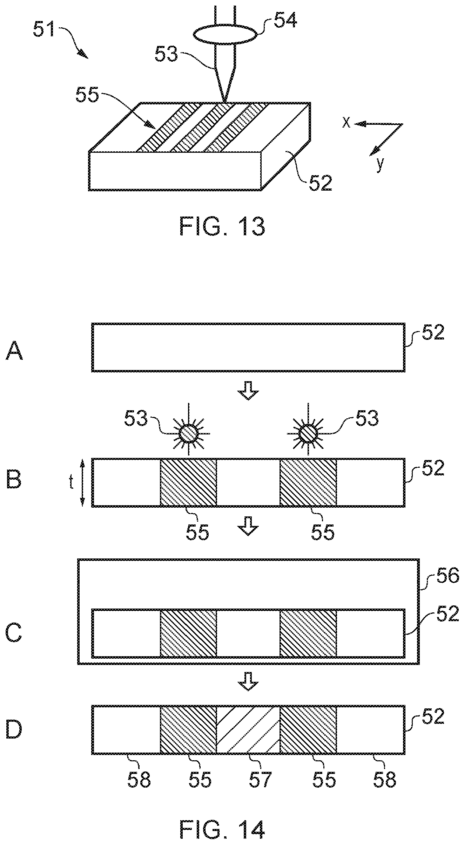

Figure 13 shows a highly simplified schematic representation of a system 51 for performing a light-writing technique. A planar substrate 52 of paper (or other porous material) impregnated with a light-sensitive substance in the form of a photopolymer is provided. A laser (not shown) delivers a light beam 53 which is focussed using one or more mirrors or lenses 54 and directed onto the surface of the substrate 52. Relative translation in the X and Y directions (in the plane of the substrate) between the substrate surface and the light beam is used to trace or write a pattern of lines on the substrate 52. The light delivers energy into the substrate and acts to cross-link/polymerise the photopolymer (changing it from its first state into its second state) below the sites or areas (the written lines) of light exposure, which creates a series of solidified polymer lines (walls or barriers) within the substrate. In this example, a set of three parallel lines 55 has been written.

Figure 14 shows a schematic illustration of various steps in this process, as side views of the substrate. In step A (impregnation), the porous substrate 52 is impregnated with the light-sensitive curable photopolymer (also variously called resist, resin or adhesive), by soaking it in a solution of the photopolymer. In step B (exposure), the focussed light beam 53 is scanned directly over the surface of the substrate 52 to write the required pattern of lines, in this example two lines. Under each line, a volume of the substrate which has been exposed to a sufficient amount of light energy experiences polymerisation of the photopolymer as it is changed from the first, liquid, state to the second (solid, less permeable, hydrophobic and/or less soluble) state so that two walls of solid polymer 55 are formed. The walls extend through the thickness t of the substrate, from the top surface to the bottom surface. In step C (development), the substrate 52 is developed by immersion in a solvent 56 which acts to remove the unpolymerised substance (those volumes of photopolymer still in the first state) from the substrate 52, leaving the plain, untreated and hence hydrophilic substrate material in those parts of the substrate that have not been exposed to the light beam. Step D shows the finished substrate (which may be a finished device, or may require further manufacturing steps to produce the device). The two solid walls 55 form boundaries for a region of the substrate between the walls 57 which has not been polymerised so now comprises plain substrate material, and which is hence the fluid flow channel 57 since the walls 55 will act to confine fluid introduced into that region so that it flows along the channel by wicking. On the other sides of the walls 55, the substrate is also plain untreated substrate material 58.

Although Figure 14 shows an example where the radiation-sensitive substance is polymerised through the entire thickness of the substrate, it is also possible to control the properties of the laser beam (e.g. the speed of relative translation between the substrate and the beam, or the energy density or fluence of the beam) so that the substance is converted to the second state through only part of the thickness of the substrate. It is also possible to form a semi-permeable barrier that extends all the way through the porous substrate. The partially-permeable barrier can be formed, for example, by applying lower energy so that the substance is not completely polymerised, forming a wall or barrier of more polymerised material with some "holes" of less polymerised material which can be removed with a solvent. This can be useful for forming structures for controlling the rate at which fluid flows through the substrate, or for filtering particles within the fluid.

In the example of Figure 14, the substrate is completely impregnated with the radiation-sensitive substance. However, it is also possible to deposit the radiation-sensitive substance in certain patterns, e.g. patterns of walls or barriers, before applying the radiation to the substrate; in one embodiment, the substrate may be exposed in general to radiation; in another embodiment, a radiation beam may follow the same pattern. For example, as shown in Figure 15, a deposition head may be used to deposit the radiation-sensitive substance, and the relative motion between the substrate and the deposition head and laser beam may cause the deposition head and laser to travel across the substrate one after the other. Hence, the pattern of walls and barriers may be defined at least in part by the deposition of the substance, rather than the application of radiation. One advantage is that areas of the substrate which are not deposited with the substance retain the inherent properties of the substrate, such as the porosity or natural wicking ability of the substrate. Also, less radiation-sensitive substance is needed, and there is less excess substance to be removed in a development stage. In some examples, the development stage may be eliminated entirely since all the radiation-sensitive substance can be converted to a less permeable state, which is both more efficient, and avoids problems associated with residual radiation-sensitive substance remaining in the substrate. In other examples, there may still be some excess radiation-sensitive substance in the more permeable state, for instance following deposition the radiation-sensitive substance may have spread outwards while soaking through the substrate, so that there is some substance beyond the location of the deposited surface pattern, and depending on the width and positioning of the beam of radiation used this may lead to some parts of the substance not being converted into the less permeable state, in which case some development may still be required. Nevertheless, the amount of excess radiation-sensitive substance to be removed can be greatly reduced.

Hence, as shown in Figure 16, by forming a partial barrier 60 extending through part of the thickness of the substrate, a constricted portion 62 of the fluid flow channel 6 can be formed in the LFD 4, which is restricted in the thickness dimension (tₛ < t, where t is the thickness of the substrate), rather than being restricted in the width dimension (the channel width w can be constant throughout the channel 6 in the example of Figure 16). Alternatively, other embodiments could limit the constricted portion in both the thickness and width dimensions.

Clearly, a wide range and variety of flow devices and structures on porous substrates can be fabricated using the methods described herein. As mentioned, the shape of the fluid-containing regions can vary, e.g. including channels along which fluid may flow, which may have a fixed or a varying width and follow straight, spiral or serpentine paths. Reservoirs, wells and the like may also be used. These may be self-contained for confining received fluid (in the manner of a microtiter plate, for example), or may be connected to other regions by one or more channels, to give a fluid flow network. A single substrate may include more than one network, or multiple self-contained regions. Any number of sample or analyte introduction locations can be connected to any number of test zones or locations via any number, shape and pattern of flow channels.

As discussed above, it is possible to form walls which are fully impermeable to the fluid and extend through substantially the full thickness of the substrate, by depositing radiation-sensitive substance which extends right through the substrate, and exposing that substance to sufficient light energy to solidify it through the full substrate thickness. However, the LDW technique is not so limited, and may also be used to form barriers within flow channels and other fluid-containing regions which are partially permeable. Such barriers, being volumes of solidified polymer within a flow channel which extend through only part of the substrate thickness, or which extend through the full thickness but are in a partially permeable state, can be added to any of the channels or other network structures to control flow within the channels, where the barriers may have a substantially constant thickness to reduce fluid speed, or may have a thickness variation along the flow direction to produce one-directional flow. Varying depth barrier profiles can also be used for delay alone. Thus, the barrier, by forming an obstacle within a flow channel, acts to impede the flow of fluid past the barrier. According to embodiments, partial barriers of this type can be formed by depositing radiation-sensitive substance onto the substrate within a region intended to receive fluid (such as between the walls of a channel) and applying sufficient light energy to cause hardening through only part of the substrate thickness. This can be achieved by depositing the radiation-sensitive substance such that it extends only partly through the substrate thickness and then fully solidifying all of that substance by light exposure, or by depositing the radiation-sensitive substance to extend through most or all of the substrate thickness and solidifying only part of the thickness of the substance by applying a reduced amount of radiation. The former technique may be preferred since it may leave less residual unsolidified polymer within the fluid network. Alternatively, the porosity of a barrier can be controlled depending on the radiation energy used, to provide varying degrees of delay to fluid flow. A partial barrier can be defined by a particular line of the deposited pattern that occupies an area within a region intended for fluid, so that both full thickness walls and partial barriers can be formed by a single application of the LDW method. Alternatively, the method may be used to create partial barriers only, perhaps within an existing fluid network already fabricated in a substrate by any technique.

The radiation-sensitive substance may be deposited on the surface of the substrate and then may spread through the thickness of the substrate so that when converted to the second state under the application of radiation, the radiation-sensitive substance forms less permeable walls or barriers within the substrate.

However, it is also possible to form structures on the surface of the substrate using the LDW technique. For example, when the radiation-sensitive substance is deposited, while some of the substance may soak through into the substrate, a film of substance may remain on the surface of the substrate for some time after depositing the substance. If the radiation is applied while there is still a pattern of substance remaining on the surface of the substrate, then when the radiation is applied the surface substance will also be converted to the second state, forming "bumps" which project above the surface of the substrate. This can be useful, for example, when wells or channels are formed within the substrate for containing or guiding fluid, as the surface bumps can prevent fluid spilling out of the wells or channels over the surface of the substrate.

A colour change caused by the reaction between analyte and reagent at a test site on a device can be employed to encode the result of the test such that it can be read automatically, or only understood by a healthcare professional. For example, the substrate may be printed with a one- or two-dimensional pattern similar to a bar code or a QR code. A colour-change reagent may be embedded at one or more locations within the pattern in a colourless manner; these are connected to an analyte introduction site elsewhere on the substrate by one or more channels. Analyte introduced onto the substrate will flow to the test location(s), and possibly cause a colour change at the location(s) depending on a positive or negative test. The colour change will alter the shape of the pattern, which can then be read by a hand-held scanner or photographed by a mobile telephone or tablet camera for communication to a remote diagnostic facility or website, and interpreted accordingly. Other information, such as type of test, and patient identity, could be encoded in the test pattern also, to further facilitate automated testing.

Fluid flow devices according to embodiments of the invention are not limited to medical diagnostics. Biological and chemical sensors in lateral flow test formats for sample testing are also required in fields including veterinary medicine, the food, beverage, water and pharmaceutical industries, agriculture, environmental sensing, and defence & security applications. The invention can provide devices for use in all of these fields, and any other requiring fluid sample diagnostics or testing.

A variety of materials may be employed as a substrate. Typically, the substrate will be planar, such as a sheet or layer of material. Paper has been found to be of particular interest, since it is readily available in a range of thicknesses, densities, porosities and colours, is inexpensive, can be easily cut to size, can be printed with instructions, directions and indicia, can be folded, and is lightweight. However, other porous materials may be used instead, such as cardboard, or woven and non-woven fabrics made from natural or synthetic fibres and combinations thereof. If the LDW method is used, the substrate material should be able to withstand exposure to the required radiation energy density without suffering unacceptable ablation or other damage, and be able to undergo any subsequent processing steps needed to produce the finished device. A material which displays these characteristics may be used as the substrate. The properties and characteristics of potential substrate materials can be compared when selecting a potential substrate for a particular device. A property of particular interest is the density of the material as expressed through its internal structure, or its pore sizes, since this will affect the size of the solid structures which may be written into it. A material with an open structure (such a large-grained paper with wide grain spacing) may have a minimum barrier width that is able to fully contain fluid within a region, so that thinner structures cannot be used on such a substrate. This may affect the overall minimum device size which is achievable.

Another substrate material of special interest is nitrocellulose. Nitrocellulose membranes have particular application in point-of-care biosensor devices (such as pregnancy tests) since the material has a range of advantages. It has a high binding affinity for proteins, it produces only a low background signal, and is compatible with a variety of detection methods including chemiluminescent, chromogenic and fluorescent techniques. Also, the manufacture of nitrocellulose, which is well-established at the industrial scale, can be controlled to produce pores of specific sizes which are large enough to allow fluid flow.

Other examples of substrates may include sintered materials such as sintered glass or sintered plastics.

Some substrate materials may have wicking ability, which can be useful because this allows a fluid to be drawn along a channel in the required fluid flow and the radiation-sensitive substance can be drawn down through the substrate thickness in the deposition stage.

Other materials may not have an inherent wicking ability, and instead the fluid flow may be controlled by the application of an external factor, such as an electric field or exposure to radiation. This can be useful for example because the fluid flow device may take a fluid sample at the point of care, but then may need to be inserted into a device reader or other apparatus in order to carry out the actual analysis of the sample. If there is a delay in transferring the device to the reader, then if the fluid naturally flows through the substrate, by the time the device reaches the reader the fluid may already have flowed through the fluid flow network and out of the device preventing appropriate analysis. This problem can be avoided if the fluid does not naturally flow through the substrate unless an external factor is applied.

For example, if the fluid is an ionic fluid, an electric field could be applied by the reader to trigger the flow of fluid.

Alternatively, if the fluid is not itself ionic, flow of fluid may be controlled by varying the wetting properties of the structures in the fluid flow network. For example, the wetting properties (e.g. hydrophobicity) of some materials may change when an electric field is applied or when electromagnetic radiation is applied. Such a material may be injected to coat the insides of the structures formed by the method above, so that they retain the fluid in an initial state and become more hydrophobic on application of the electric field or exposure to electromagnetic radiation, so that fluid starts to flow.

In some examples, different types of substrates can be joined to form a composite substrate, and then form the structures on the composite substrate using the method described above. This can be useful for providing different fluid flow rates in different parts of the composite substrate, for example.

The radiation applied in the LDW method may be any form of radiation capable of changing the deposited material from a first state to a second state, where the substance is less permeable, more solid and/or less soluble in one of the first state and the second state than the other.

In one embodiment, the radiation comprises electromagnetic radiation. Electromagnetic radiation of any desired wavelength may be used. Preferred forms of radiation include ultraviolet radiation (typically defined as electromagnetic radiation having a wavelength of 20 to 400 nm) and visible light (typically defined as electromagnetic radiation having a wavelength of 400 to 700 nm).

Other sources of radiation may include ion beams, electron beams, and ultrasound, for example.

Lasers provide convenient sources of radiation, since their beams can be focussed to a small spot size, a range of wavelengths are available, power can be easily adjusted, and beam scanning is readily implementable. However, other radiation sources may be used if preferred. For example, the radiation source may be a supercontinuum source, one or more light emitting diodes, or other source which is sufficiently bright and of the proper wavelength to produce the required transformation of the radiation-sensitive substance into the hardened state. The wavelength may also be selected having regard to the properties of any reagent to be provided in the fluid flow device, as discussed above.

The radiation spot as exposed onto the surface of the substrate can be produced by any arrangement which gives a spot of sufficient intensity or energy density to induce the state change in the radiation-sensitive substance. Often, this will be an arrangement such as focussing or imaging of the incident light beam which substantially reduces the spot size (while giving a spot of the required dimension for the line which is to be polymerised) so as to give a significant increase in the energy density of the beam. An unfocussed beam may be used instead, however. The light beam will generally be exposed directly onto the substrate surface to define the writing spot on the substrate surface, subject to any lenses, mirrors and the like used to form, shape and direct the light beam into the required spot size and shape. "Directly" indicates that there is no intervening mask or similar, such as is required in lithographic techniques.

The light source may provide a continuous emission of light or a pulsed emission, for example a laser source that produces regular or irregular pulses with durations on the nanosecond, picosecond or femtosecond scale. The terms "light beam", "beam", and "beam of light" are used to include both the continuous and pulsed alternatives.

It is also possible to apply more than one source of radiation to the same device. For example, sources of radiation of different types may be provided (e.g. ambient light and laser light, or ion beam radiation and laser light). Also, the different sources of radiation could provide radiation of different wavelengths, frequencies or energy density, for example. This can be used to provide further control of the formation of structures in the device. For example, the different sources may provide different depths or degrees of conversion of the radiation-sensitive substance from the first state to the second state, which can provide structures with different permeability to the fluid. For example, one source of radiation (e.g. a first laser) could be used to form walls and another source (e.g. a laser of different frequency or energy) could form barriers. In another example, the different types of radiation could for example correspond to different types of laser such as a pulsed laser and a continuous wave laser (see for example the experimental work below where pulsed and c.w. lasers can be used to form different kinds of barrier).

In some cases, the different sources of incident radiation may be applied in series so that a first source is applied initially, and later a second source is applied.

It is also possible to use simultaneous sources of radiation in parallel, so that multiple structures or barriers can be created at the same time. For example, a beam of radiation could be split into multiple beams each of which may be used to write a line, wall or barrier.

While the examples shown above show LFDs with a single layer of substrate and fluid flow constrained within 2 dimensions, it is also possible to provide multi-layer LFDs comprising a number of layers of substrate stacked on top of each other. In some cases, each layer could provide an entirely independent network of fluid flow channels. For example, a standard LFD 2 and constricted flow LFD 4 could be stacked on top of each other to provide increased dynamic range in a similar way to the examples in Figures 10-12, or different tests could be applied to the sample in different layers. Hence, the constricted flow LFD channel 6 discussed above could be formed in one layer of a multi-layer LFD, but other layers could have other networks of channels. An impermeable barrier layer could be provided between neighbouring layers of porous substrate to block passage of fluid from one layer to the other. Alternatively, some multi-layer LFD may also implement 3D flow of fluid, with fluid able to pass between layers at some points along the plane of the substrate (e.g. by having holes in selected locations of the barrier layer). This could be used to implement more complex networks of channels extending in 3 dimensions through a stack of multiple layers. In this case, the constricted flow channels could be formed in any one or more of the layers.

Although illustrative embodiments of the invention have been described in detail herein with reference to the accompanying drawings, it is to be understood that the invention is not limited to those precise embodiments, and that various changes and modifications can be effected therein by one skilled in the art without departing from the scope of the invention as defined by the appended claims.

## Claims

1. A lateral flow diagnostic device (4) comprising:
a substrate (5) of porous material;
channel boundaries (11) defining a first fluid flow channel (CL1) within the substrate; and
a first test site (10) disposed at a predetermined location along the first fluid flow channel, the first test site comprising an analyte detection substance for detecting presence of a target analyte in fluid flowing along the first fluid flow channel; in which:
the first fluid flow channel comprises a constricted portion (Lc) with a smaller cross-sectional area than another portion of the fluid flow channel; and
the first test site is disposed within the constricted portion of the first fluid flow channel;
the lateral flow diagnostic device comprising a plurality of fluid flow channels, at least two of the fluid flow channels comprising a respective test site comprising the same analyte detection substance, wherein the at least two fluid flow channels include at least:
the first fluid flow channel comprising said constricted portion; and
one of:
a second fluid flow channel (CL2) comprising a constricted portion with a second cross-sectional area different from a first cross-sectional area of the constricted portion of the first fluid flow channel, or
a second fluid flow channel without said constricted portion and having a larger cross-sectional area than the constricted portion of the first fluid flow channel.

2. The lateral flow diagnostic device according to claim 1, in which:
the first fluid flow channel comprises a fluid inlet for introducing fluid into the fluid flow channel; and
an initial portion of the first fluid flow channel between the fluid inlet and the constricted portion has a larger cross-sectional area than the constricted portion.

3. The lateral flow diagnostic device according to any of claims 1 or 2, in which:
a subsequent portion of the first fluid flow channel lying beyond the constricted portion in a direction of fluid flow through the fluid flow channel has a larger cross-sectional area than the constricted portion.

4. The lateral flow diagnostic device according to any preceding claim, comprising a control site (12) disposed within the first fluid flow channel comprising a control substance for detecting presence of a substance indicative of validity of a test performed at the first test site.

5. The lateral flow diagnostic device according to claim 4, in which the control site is within the constricted portion of the first fluid flow channel.

6. The lateral flow diagnostic device according to any preceding claim, in which a maximum width, c, of the constricted portion of the first fluid flow channel in a direction parallel to the plane of the substrate is narrower than a minimum width, w, of the other portion of the first fluid flow channel in the direction parallel to the plane of the substrate.

7. The lateral flow diagnostic device according to claim 6, in which:
the ratio w/c is greater than or equal to 1.2, and/or
w - c is greater than 0.8 mm.

8. The lateral flow diagnostic device according to any preceding claim, in which a maximum thickness of the constricted portion of the first fluid flow channel in a direction perpendicular to the plane of the substrate is smaller than a minimum thickness of the other portion of the first fluid flow channel in the direction perpendicular to the plane of the substrate.

9. The lateral flow diagnostic device according to any preceding claim, in which a length of the constricted portion along the first fluid flow direction is greater than or equal to 4 mm.

10. The lateral flow diagnostic device according to any preceding claim, in which the channel boundaries in the constricted portion of the first fluid flow channel comprise one of:
the edges of the substrate, or
structures formed within the substrate, where the structures are less porous than the material of the substrate.

11. The lateral flow diagnostic device according to any preceding claim, in which the substrate comprises one of:
paper;
nitrocellulose; and
sintered glass.

12. A method for manufacturing a lateral flow diagnostic device (4), comprising:
forming channel boundaries (11) defining a first fluid flow channel (CL1) within a substrate of porous material, wherein the first fluid flow channel comprises a constricted portion (Lc) with a smaller cross-sectional area than another portion of the first fluid flow channel; and
forming a first test site (10) disposed at a predetermined location along the first fluid flow channel, the first test site comprising an analyte detection substance for detecting presence of a target analyte in fluid flowing along the first fluid flow channel, wherein the first test site is formed within the constricted portion of the first fluid flow channel, and
the lateral flow diagnostic device comprises a plurality of fluid flow channels, at least two of the fluid flow channels comprising a respective test site comprising the same analyte detection substance, wherein the at least two fluid flow channels include at least:
the first fluid flow channel comprising said constricted portion; and
one of:
a second fluid flow channel (CL2) comprising a constricted portion with a second cross-sectional area different from a first cross-sectional area of the constricted portion of the first fluid flow channel, or
a second fluid flow channel without said constricted portion and having a larger cross-sectional area than the constricted portion of the first fluid flow channel.

13. The method of claim 12, in which the channel boundaries are formed by applying radiation (53) onto a radiation-sensitive substance impregnated in the substrate, to change the radiation-sensitive substance from a first state to a second state through at least part of the thickness of the substrate.

14. The method of claim 13, comprising depositing the radiation-sensitive substance at selected locations on the substrate corresponding to the positions of the channel boundaries.

15. The method of any of claims 13 or 14, in which the radiation-sensitive substance is at least one of less permeable to fluid, more solid, or less soluble to a developer in the second state than in the first state.

## Patentansprüche

1. Lateral-Flow-Diagnosegerät (4), umfassend:
ein Substrat (5) aus porösem Material;
Kanalgrenzen (11), die einen ersten Fluidflusskanal (CL1) innerhalb des Substrats definieren; und
eine erste Teststelle (10), die an einem vorbestimmten Ort entlang des ersten Fluidflusskanals angeordnet ist, wobei die erste Teststelle eine Analytdetektierungssubstanz zum Detektieren der Anwesenheit eines Zielanalyten in Fluid umfasst, welches den ersten Fluidflusskanal entlang fließt;
wobei:
der erste Fluidflusskanal einen verengten Abschnitt (Lc) mit einer kleineren Querschnittfläche als ein anderer Abschnitt des Fluidflusskanals umfasst; und
die erste Teststelle innerhalb des verengten Abschnitts des ersten Fluidflusskanals angeordnet ist;
wobei das Lateral-Flow-Diagnosegerät eine Vielzahl von Fluidflusskanälen umfasst, wobei mindestens zwei der Fluidflusskanäle eine jeweilige Teststelle umfassen, welche die gleiche Analytdetektierungssubstanz umfasst, wobei die mindestens zwei Fluidflusskanäle mindestens einschließen:
den ersten Fluidflusskanal, der den verengten Abschnitt umfasst; und einen von:
einem zweiten Fluidflusskanal (CL2), der einen verengten Abschnitt mit einer zweiten QuerSchnittfläche umfasst, die sich von einer ersten Querschnittfläche des verengten Abschnitts des ersten Fluidflusskanals unterscheidet, oder
einem zweiten Fluidflusskanal ohne den verengten Abschnitt und mit einer größeren Querschnittfläche als der verengte Abschnitt des ersten Fluidflusskanals.

2. Lateral-Flow-Diagnosegerät nach Anspruch 1, wobei:
der erste Fluidflusskanal einen Fluideinlass zum Einbringen von Fluid in den Fluidflusskanal umfasst; und
ein anfänglicher Abschnitt des ersten Fluidflusskanals zwischen dem Fluideinlass und dem verengten Abschnitt eine größere Querschnittfläche als der verengte Abschnitt hat.

3. Lateral-Flow-Diagnosegerät nach einem der Ansprüche 1 oder 2, wobei:
ein nachfolgender Abschnitt des ersten Fluidflusskanals, der hinter dem verengten Abschnitt in Richtung des Fluidflusses durch den Fluidflusskanal hindurch liegt, eine größere Querschnittfläche als der verengte Abschnitt hat.

4. Lateral-Flow-Diagnosegerät nach einem der vorhergehenden Ansprüche, umfassend eine Kontrollstelle (12), die innerhalb des ersten Fluidflusskanals angeordnet ist und eine Kontrollsubstanz zum Detektieren der Anwesenheit einer Substanz umfasst, wodurch die Validität eines an der ersten Teststelle durchgeführten Tests angegeben wird.

5. Lateral-Flow-Diagnosegerät nach Anspruch 4, wobei die Kontrollstelle innerhalb des verengten Abschnitts des ersten Fluidflusskanals liegt.

6. Lateral-Flow-Diagnosegerät nach einem der vorhergehenden Ansprüche, wobei eine Maximalbreite, c, des verengten Abschnitts des ersten Fluidflusskanals in einer Richtung parallel zu der Ebene des Substrats schmaler als eine Minimalbreite, w, des anderen Abschnitts des ersten Fluidflusskanals in der Richtung parallel zu der Ebene des Substrats ist.

7. Lateral-Flow-Diagnosegerät nach Anspruch 6, wobei:
das Verhältnis w/c größer als oder gleich 1,2 ist, und/oder
w - c größer als 0,8 mm ist.

8. Lateral-Flow-Diagnosegerät nach einem der vorhergehenden Ansprüche, wobei eine Maximaldicke des verengten Abschnitts des ersten Fluidflusskanals in einer Richtung senkrecht zu der Ebene des Substrats kleiner als eine Minimaldicke des anderen Abschnitts des ersten Fluidflusskanals in der Richtung senkrecht zu der Ebene des Substrats ist.

9. Lateral-Flow-Diagnosegerät nach einem der vorhergehenden Ansprüche, wobei eine Länge des verengten Abschnitts entlang der ersten Fluidflussrichtung größer als oder gleich 4 mm ist.

10. Lateral-Flow-Diagnosegerät nach einem der vorhergehenden Ansprüche, wobei die Kanalgrenzen in dem verengten Abschnitts des ersten Fluidflusskanals eine der folgenden umfassen:
die Ränder des Substrats, oder
Strukturen, die innerhalb des Substrats gebildet sind, wobei die Strukturen weniger porös als das Material des Substrats sind.

11. Lateral-Flow-Diagnosegerät nach einem der vorhergehenden Ansprüche, wobei das Substrat eines der folgenden umfasst:
Papier;
Nitrocellulose; und
Sinterglas.

12. Verfahren zur Fertigung eines Lateral-Flow-Diagnosegeräts (4), umfassend:
Bilden von Kanalgrenzen (11), die einen ersten Fluidflusskanal (CL1) innerhalb eines Substrats aus porösem Material definieren, wobei der erste Fluidflusskanal einen verengten Abschnitt (Lc) mit einer kleineren Querschnittfläche als ein anderer Abschnitt des ersten Fluidflusskanals umfasst; und
Bilden einer ersten Teststelle (10), die an einem vorbestimmten Ort entlang des ersten Fluidflusskanals angeordnet ist, wobei die erste Teststelle eine Analytdetektierungssubstanz zum Detektieren der Anwesenheit eines Zielanalyten in Fluid umfasst, welches den ersten Fluidflusskanal entlang fließt, wobei die erste Teststelle innerhalb des verengten Abschnitts des ersten Fluidflusskanals gebildet ist, und
wobei das Lateral-Flow-Diagnosegerät eine Vielzahl von Fluidflusskanälen umfasst, wobei mindestens zwei der Fluidflusskanäle eine jeweilige Teststelle umfassen, welche die gleiche Analytdetektierungssubstanz umfasst, wobei die mindestens zwei Fluidflusskanäle mindestens einschließen:
den ersten Fluidflusskanal, der den verengten Abschnitt umfasst; und einen von:
einem zweiten Fluidflusskanal (CL2), der einen verengten Abschnitt mit einer zweiten Querschnittfläche umfasst, die sich von einer ersten Querschnittfläche des verengten Abschnitts des ersten Fluidflusskanals unterscheidet, oder
einem zweiten Fluidflusskanal ohne den verengten Abschnitt und mit einer größeren Querschnittfläche als der verengte Abschnitt des ersten Fluidflusskanals.

13. Verfahren nach Anspruch 12, wobei die Kanalgrenzen durch Anwenden von Strahlung (53) auf eine strahlungsempfindliche Substanz gebildet werden, die in das Substrat imprägniert ist, um die strahlungsempfindliche Substanz durch mindestens einen Teil der Dicke des Substrats hindurch von einem ersten Zustand zu einem zweiten Zustand zu verändern.

14. Verfahren nach Anspruch 13, umfassend Absetzen der strahlungsempfindlichen Substanz an ausgewählten Orten auf dem Substrat entsprechend den Positionen der Kanalgrenzen.

15. Verfahren nach einem der Ansprüche 13 oder 14, wobei die strahlungsempfindliche Substanz in dem zweiten Zustand, verglichen mit dem ersten Zustand, mindestens eines von weniger fluidpermeabel, massiver oder weniger löslich in einem Entwickler ist.

## Revendications

1. Dispositif de diagnostic à écoulement latéral (4) comprenant :
un substrat (5) en matériau poreux ;
des limites de canal (11) définissant un premier canal d'écoulement de fluide (CL1) à l'intérieur du substrat ; et
un premier site de test (10) disposé à un emplacement prédéterminé le long du premier canal d'écoulement de fluide, le premier site de test comprenant une substance de détection d'analyte pour détecter la présence d'un analyte cible dans le fluide s'écoulant le long du premier canal d'écoulement de fluide ; dans lequel :
le premier canal d'écoulement de fluide comprend une partie rétrécie (Lc) avec une surface de section transversale plus petite qu'une autre partie du canal d'écoulement de fluide ; et
le premier site de test est disposé à l'intérieur de la partie rétrécie du premier canal d'écoulement de fluide ;
le dispositif de diagnostic à écoulement latéral comprenant une pluralité de canaux d'écoulement de fluide, au moins deux des canaux d'écoulement de fluide comprenant un site de test respectif comprenant la même substance de détection d'analyte, dans lequel les au moins deux canaux d'écoulement de fluide comprennent au moins :
le premier canal d'écoulement de fluide comprenant ladite partie rétrécie ; et
un parmi :
un second canal d'écoulement de fluide (CL2) comprenant une partie rétrécie avec une seconde surface de section transversale différente d'une première surface de section transversale de la partie rétrécie du premier canal d'écoulement de fluide, ou
un second canal d'écoulement de fluide sans ladite partie rétrécie et ayant une surface de section transversale plus grande que la partie rétrécie du premier canal d'écoulement de fluide.

2. Dispositif de diagnostic à écoulement latéral selon la revendication 1, dans lequel :
le premier canal d'écoulement de fluide comprend une entrée de fluide pour introduire un fluide dans le canal d'écoulement de fluide ; et
une partie initiale du premier canal d'écoulement de fluide entre l'entrée de fluide et la partie rétrécie a une surface de section transversale plus grande que la partie rétrécie.

3. Dispositif de diagnostic à écoulement latéral selon l'une quelconque des revendications 1 ou 2, dans lequel :
une partie ultérieure du premier canal d'écoulement de fluide se trouvant au-delà de la partie rétrécie dans une direction d'écoulement de fluide à travers le canal d'écoulement de fluide a une surface de section transversale plus grande que la partie rétrécie.

4. Dispositif de diagnostic à écoulement latéral selon l'une quelconque revendication précédente, comprenant un site de contrôle (12) disposé à l'intérieur du premier canal d'écoulement de fluide comprenant une substance de contrôle pour détecter la présence d'une substance indiquant la validité d'un test effectué au niveau du premier site de test.

5. Dispositif de diagnostic à écoulement latéral selon la revendication 4, dans lequel le site de contrôle se trouve à l'intérieur de la partie rétrécie du premier canal d'écoulement de fluide.

6. Dispositif de diagnostic à écoulement latéral selon l'une quelconque revendication précédente, dans lequel une largeur maximale, c, de la partie rétrécie du premier canal d'écoulement de fluide dans une direction parallèle au plan du substrat est plus étroite qu'une largeur minimale, w, de l'autre partie du premier canal d'écoulement de fluide dans la direction parallèle au plan du substrat.

7. Dispositif de diagnostic à écoulement latéral selon la revendication 6, dans lequel :
le rapport w/c est supérieur ou égal à 1,2, et/ou w - c est supérieur à 0,8 mm.

8. Dispositif de diagnostic à écoulement latéral selon l'une quelconque revendication précédente, dans lequel une épaisseur maximale de la partie rétrécie du premier canal d'écoulement de fluide dans une direction perpendiculaire au plan du substrat est inférieure à une épaisseur minimale de l'autre partie du premier canal d'écoulement de fluide dans la direction perpendiculaire au plan du substrat.

9. Dispositif de diagnostic à écoulement latéral selon l'une quelconque revendication précédente, dans lequel une longueur de la partie rétrécie le long de la première direction d'écoulement de fluide est supérieure ou égale à 4 mm.

10. Dispositif de diagnostic à écoulement latéral selon l'une quelconque revendication précédente, dans lequel les limites de canal dans la partie rétrécie du premier canal d'écoulement de fluide comprennent un parmi :
les bords du substrat, ou
des structures formées à l'intérieur du substrat, les structures étant moins poreuses que le matériau du substrat.

11. Dispositif de diagnostic à écoulement latéral selon l'une quelconque revendication précédente, dans lequel le substrat comprend un parmi :
du papier ;
de la nitrocellulose ; et
du verre fritté.

12. Procédé de fabrication d'un dispositif de diagnostic à écoulement latéral (4), comprenant :
la formation de limites de canal (11) définissant un premier canal d'écoulement de fluide (CL1) à l'intérieur d'un substrat de matériau poreux, dans lequel le premier canal d'écoulement de fluide comprend une partie rétrécie (Lc) avec une surface de section transversale plus petite qu'une autre partie du premier canal d'écoulement de fluide ; et
la formation d'un premier site de test (10) disposé à un emplacement prédéterminé le long du premier canal d'écoulement de fluide, le premier site de test comprenant une substance de détection d'analyte pour détecter la présence d'un analyte cible dans un fluide s'écoulant le long du premier canal d'écoulement de fluide, dans lequel le premier site de test est formé à l'intérieur de la partie rétrécie du premier canal d'écoulement de fluide, et
le dispositif de diagnostic à écoulement latéral comprend une pluralité de canaux d'écoulement de fluide, au moins deux des canaux d'écoulement de fluide comprenant un site de test respectif comprenant la même substance de détection d'analyte, dans lequel les au moins deux canaux d'écoulement de fluide comprennent au moins :
le premier canal d'écoulement de fluide comprenant ladite partie rétrécie ; et
un parmi :
un second canal d'écoulement de fluide (CL2) comprenant une partie rétrécie avec une seconde surface de section transversale différente d'une première surface de section transversale de la partie rétrécie du premier canal d'écoulement de fluide, ou
un second canal d'écoulement de fluide sans ladite partie rétrécie et ayant une surface de section transversale plus grande que la partie rétrécie du premier canal d'écoulement de fluide.

13. Procédé selon la revendication 12, dans lequel les limites de canal sont formées par application d'un rayonnement (53) sur une substance sensible au rayonnement imprégnée dans le substrat, pour faire passer la substance sensible au rayonnement d'un premier état à un second état à travers au moins une partie de l'épaisseur du substrat.

14. Procédé selon la revendication 13, comprenant le dépôt de la substance sensible au rayonnement à des emplacements sélectionnés sur le substrat correspondant aux positions des limites de canal.

15. Procédé selon l'une quelconque des revendications 13 ou 14, dans lequel la substance sensible au rayonnement est moins perméable aux fluides et/ou plus solide et/ou moins soluble dans un révélateur dans le second état que dans le premier état.
